# EUROPEAN PATENT APPLICATION

(11) **EP 2 305 285 A1**
(43) Date of publication of application: **06.04.2011**
(21) Application number: 09012330.8
(22) Date of filing: 29.09.2009
(51) Int. Cl.: A61K 38/17, A61K 31/573, A61K 45/06, A61P 9/10

(54) **Means and methods for treating ischemic conditions**

(71) Applicant: Julius-Maximilians-Universität Würzburg, 97070 Würzburg (DE)
(72) Inventor: Förster, Carola, 97070 Würzburg (DE)
(74) Representative: Vossius & Partner

(57) **Abstract**

The present invention relates to a combination of (a) at least one glucocorticoid; and (ba) at least one proteasome inhibitor and/or (bb) at least one nucleic acid encoding a glucocorticoid receptor, said glucocorticoid receptor being resistant to proteasomal degradation, for use in the treatment or prevention of ischemic disorders of the central nervous system. Furthermore provided is at least one proteasome inhibitor and/or at least one nucleic acid encoding a glucocorticoid receptor, said glucocorticoid receptor being resistant to proteasomal degradation, for use in treating or preventing glucocorticoid resistance in ischemic disorders of the central nervous system.

## Description

This invention relates to a combination of (a) at least one glucocorticoid; and (ba) at least one proteasome inhibitor and/or (bb) at least one nucleic acid encoding a glucocorticoid receptor, said glucocorticoid receptor being resistant to proteasomal degradation, for use in the treatment or prevention of ischemic disorders of the central nervous system.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Disruption of the blood-brain barrier (BBB) and concomitant edema formation are pathological hallmarks of various disorders of the central nervous system. The BBB is formed by brain capillary endothelial cells (Rubin (1999)). Tight junctions are important structural components of the BBB which seal the paracellular space between brain microvascular endothelial cells and thereby restrict paracellular permeability (Förster (2008), Wolburg (2002)). Among the tight junction proteins expressed at the BBB, the transmembrane proteins occludin and claudin-5 have been shown to be critical for sealing the paracellular space (D'Atri (2002)) and degradation of these proteins is involved in BBB breakdown during multiple sclerosis, meningitis or brain neoplasm (rev. in Förster (2008)). The impact of focal cerebral ischemia on the expression of tight junction proteins has only recently begun to be addressed. Localization of claudin-5 in the plasma membrane and expression of claudin-5 protein was altered under hypoxic conditions in mouse brain bEND.3 cells (Koto (2007)). This was accompanied by a decrease in transendothelial resistance. Kago and co-workers (Kago (2006)) investigated the expression of occludin and zonula occludens (ZO)-1 in isolated brain capillaries ex vivo and found downregulation after microsphere-induced cerebral embolism.

While glucocorticoids (GC) potently stabilize the BBB and diminish tissue edema in neuroinflammatory diseases (e.g. multiple sclerosis) or brain tumors, these substances are ineffective or even harmful when applied for the treatment of acute ischemic stroke (Norris (2004), Norris (1986), Poungvarin (2004), De Reuck (1988), Kumar (1989)). The biological effects of glucocorticoids are mediated by the glucocorticoid receptor (GR) (Beato (1989)). The GR is a member of the nuclear receptor superfamily and binds to specific DNA sequences (glucocorticoid-response element, GRE) in the 5'-flanking region of target genes thereby activating gene transcription (Beato (1989), Stoll (2000)). It is expressed in a wide range of tissues including vascular endothelium, and GR expression levels correlate with its transcriptional activity.

The ubiquitin-proteasome system controls the intracellular levels of short-lived and regulatory proteins, which are important for a variety of basic cellular processes.

WO 98/35691 A1 describes methods for treating an ischemic injury comprising the administration of an inhibitor of NF-_{K}B activation. Envisaged inhibitors include proteasome inhibitors. The notion underlying the use of proteasome inhibitors is that degradation of the inhibitory protein IκB-α is to prevented, thereby down-regulating NF-κB signalling. In a preferred embodiment, further agents may be combined with the inhibitor of NF-κB activation, wherein the further agent may be a (further) inhibitor of NF-κB activation, agents which inhibit proinflammatory cytokines, agents which act to reperfuse or oxygenate tissues, steroids, antiedema drugs, thrombolytics etc. There is no experimental evidence of any of the mentioned combination therapies. Moreover, there is no recognition of the class of glucocorticoids, let alone of the combination of a glucocorticoid with a proteasome inhibitor, and in particular not of the problem of glucocorticoid insensitivity in ischemic conditions of the central nervous system. As described above, the current perception is that glucocorticoids are ineffective in the treatment of ischemic conditions of the central nervous system, and no remedy for this ineffectiveness is being suggested.

In view of the limitations of the methods described in the prior art, the technical problem underlying the present invention was therefore the provision of alternative or improved means and methods for treating ischemic conditions.

Accordingly, this invention relates to a combination of (a) at least one glucocorticoid; and (ba) at least one proteasome inhibitor and/or (bb) at least one nucleic acid encoding a glucocorticoid receptor, said glucocorticoid receptor being resistant to proteasomal degradation, for use in the treatment or prevention of ischemic disorders of the central nervous system.

The term "combination" in conjunction with the recited treatment refers to the combined administration of at least two of the recited agents to a patient in need thereof. To explain further, the following combinations are envisaged: (i) at least one glucocorticoid and at least one proteasome inhibitor; (ii) at least one glucocorticoid and at least one nucleic acid as defined above; (iii) at least one glucocorticoid, at least one proteasome inhibitor and at least one nucleic acid as defined above. It is preferred to combine at least one glucocorticoid and at least one proteasome inhibitor. It is more preferred to combine one glucocorticoid and one proteasome inhibitor. Preferred glucocorticoids and preferred proteasome inhibitors are described below.

It is furthermore envisaged that at least one further pharmaceutically active agent is included in the combination. In a preferred embodiment, the combination does not include any such further pharmaceutically active agent.

The term "glucocorticoid" is known in the art and refers to a subclass of steroid hormones the members of which are **characterized in that** they bind the glucocorticoid receptor. The use of glucocorticoids in medicine is well established. They are used to treat diseases that are caused by an overactive immune system, such as allergies, asthma, autoimmune diseases and sepsis. They also interfere with some of the abnormal mechanisms in cancer cells, so they are used in high doses to treat cancer. In the latter case, glucocorticoids are used to drive cells, in particular malignant cells, into apoptosis. This effect is fundamentally different from the effect of glucocorticoids as exploited by the present invention which is confining damage to cells, the damage being caused by ischemia. As stated above, the art advises against the use of glucocorticoids in the treatment of ischemic conditions of the central nervous system. Preferred glucocorticoids are described further below.

The term "proteasome" is known in the art and refers to a macromolecular assembly capable of proteolytically degrading polypeptides and proteins. Proteasomes are found in all eukaryotes. Proteasomes are part of a major mechanism by which cells regulate the concentration of particular proteins and degrade misfolded proteins. The degradation process yields peptides of about seven to eight amino acids long, which can then be further degraded into amino acids and used in synthesizing new proteins. Proteins are tagged for degradation by a small protein called ubiquitin. The tagging reaction is catalyzed by enzymes called ubiquitin ligases. Once a protein is tagged with a single ubiquitin molecule, this is a signal to other ligases to attach additional ubiquitin molecules. The result is usually a polyubiquitin chain that is subsequently bound by the proteasome, allowing it to degrade the tagged protein. Structure and constituent proteins of the proteasome are known in the art and described, for example, in Finley (2009). In brief, the proteasome as a whole, also referred to as 26S-proteasome holoenzyme, typically comprises two 19S regulatory particles and one 20S catalytic core. The 20S catalytic core contains 14 distinct subunits: α1, α2, α3, α4, α5, α6, α7, β1, β2, β3, β4, β5, β6, β7. A 19S regulatory particle (19 subunits in total) contains lid and base subcomplexes. The lid subcomplex contains 9 subunits: PSMD3, PSMD12, PSMD11, PSMD6, PSMD7, PSMD4, PSMD14, PSMD8, and PSMD9, and the base subcomplex contains 10 subunits: PSMC2, PSMC1, PSMC4, PSMC6, PSMC3, PSMC5, PSMD2, PSMD1, PSMD4, and ADRM1

A "proteasome inhibitor" is a compound which interferes with the above described capability of the proteasome to degrade polypeptides and proteins. Generally speaking, the term "inhibitor" designates a compound lowering the activity of a protein, preferably by performing one or more of the following effects: (i) the transcription of the gene encoding the protein to be inhibited is lowered, (ii) the translation of the mRNA encoding the protein to be inhibited is lowered, (iii) the protein performs its biochemical function with lowered efficiency in presence of the inhibitor, and (iv) the protein performs its cellular function with lowered efficiency in presence of the inhibitor. In this context, the term "protein" refers to any of the constituent proteins of the proteasome.

Compounds falling in class (i) include compounds interfering with the transcriptional machinery and/or its interaction with the promoter of said gene and/or with expression control elements remote from the promoter such as enhancers. Compounds of class (ii) comprise antisense constructs and constructs for performing RNA interference well known in the art. Compounds of class (iii) interfere with molecular function of the protein to be inhibited, in case of the proteasome preferably with its enzymatic activity. Accordingly, active site binding compounds are envisaged. More preferred are compounds specifically binding to an active site of the proteasome. Also envisaged are compounds binding to or blocking substrate binding sites of the proteasome as are compounds binding to or blocking binding sites involved in proteasome assembly. Class (iv) includes compounds which do not necessarily directly bind to the proteasome, but still interfere with proteasome activity, for example by binding to and/or inhibiting the function or inhibiting expression of members of a pathway which comprises the proteasome. These members may be either upstream or downstream of the proteasome within said pathway. Preferred members are members of the ubiquitination pathway and are further detailed below.

In a preferred embodiment, the inhibitor is a low molecular weight compound. Low molecular weight compounds are compounds of natural origin or chemically synthesized compounds, preferably with a molecular weight between 100 and 1000, more preferred between 200 and 750, and even more preferred between 300 and 600. Preferred low molecular weight compound are small organic molecules. An organic molecule is a molecule comprising a carbon skeleton an optionally one or more heteroatoms, preferably selected from O, N, S and P.

The efficiency of the inhibitor can be quantitized by comparing the level of activity in the presence of the inhibitor to that in the absence of the inhibitor. For example, as an activity measure may be used: the change in amount of mRNA formed, the change in amount of protein formed, the change in amount of substrate converted or product formed, and/or the change in the cellular phenotype or in the phenotype of an organism.

In a preferred embodiment, the level of activity is less than 90%, more preferred less than 80%, 70%, 60% or 50% of the activity in absence of the inhibitor. Yet more preferred are inhibitors lowering the level down to less than 25%, less than 10%, less than 5%, less than 1%, less than 0,1%, less than 0,01% or less than 0,001% of the activity in absence of the inhibitor.

Preferred structural classes of proteasome inhibitors are described further below.

The intended use of the nucleic acid according to the main embodiment falls into the domain of gene therapy. Gene therapy is the introduction of nucleic acids encoding a beneficial gene product into an individual in need of the beneficial effects elicited by said gene product. The typical scenario is an individual carrying a deleterious mutation in the gene at issue and receiving an intact form of the gene. In the context of the present invention, however, it is the wild type glucocorticoid receptor which has disadvantageous properties in that it is amenable to proteasomal degradation; see further below. By making available a glucocorticoid receptor which is resistant to proteasomal degradation, this deficiency is overcome.

Constructs for gene therapy are well known in the art and familiar to the skilled person. Constructs for gene therapy generally comprise (i) the therapeutic agent which is a nucleic acid and which usually is to be delivered to a target site, target cell and/or target tissue and, optionally, (ii) an agent which enables or facilitates delivery to said target site, target cell and/or target tissue such that the therapeutic effect may occur, is enhanced, or occurs predominantly or only at the target site. For example, the nucleic acid to be delivered may be comprised in a vector, preferably a viral vector. Viral vectors for gene therapy include retroviruses, adenoviruses, and adeno-associated viruses. Furthermore, many vectors have been developed in which the endogenous viral envelope proteins have been replaced by either envelope proteins from other viruses, or by chimeric proteins. Such chimera consist of those parts of the viral protein necessary for incorporation into the virion as well as sequences meant to interact with specific host cell proteins. Viruses in which the envelope proteins have been replaced as described are referred to as pseudotyped viruses. The above described procedure is also referred to as envelope protein pseudotyping of viral vectors. Another type of constructs for gene therapy are lipoplexes and polyplexes. A lipoplex is a liposome complexed with nucleic acid. Polyplexes are complexes of polymers, preferably cationic polymers with nucleic acid. Lipoplexes and polyplexes protect the nucleic acid from undesirable degradation during the transfection process.

Under certain conditions component (i) alone may suffice and constitute the construct for gene therapy according to the invention. Examples are naked nucleic acids, preferably comprised in buffered solution.

The term "nucleic acid" as used herein refers to all forms of nucleic acids, in particular to DNA, such as cDNA or genomic DNA, and RNA. It is understood that the term "RNA" as used herein comprises all forms of RNA including mRNA.

The term "glucocorticoid receptor" is well established in the art and discussed in the introductory section herein above. The glucocorticoid receptor (GR), also known as NR3C1 (nuclear receptor subfamily 3, group C, member 1), is the receptor that glucocorticoids bind to. The glucocorticoid receptor according to item (bb) of the main embodiment is modified as compared to the wild type receptor in that it is not, or only to a reduced degree, amenable to proteasomal degradation. A reduced degree of degradability includes less than 25%, less than 10%, less than 5%, less than 1%, less than 0,1 %, less than 0,01% or less than 0,001 % degraded GR as compared to wild type GR, preferably of the same species, degraded under the same conditions. Nucleic acid and protein sequence of the glucocorticoid receptor from a plurality of species, including mammalian species, are available from the sequence databases available at, for example, the National Center for Biotechnology Information (NCBI) at the NIH and the European Bioinformatics Institute (EBI) of the EMBL. Just to give an example, entry P04150 (GCR_HUMAN) of the SwissProt database (version 152 of September 1, 2009) provides a curated annotated amino acid sequence of the human glucocorticoid receptor. The term "ischemic disorder" is known in the art and refers to conditions arising from a restriction in blood supply, generally due to factors in the blood vessels, with resultant damage or dysfunction of tissue.

The term "central nervous system", abbreviated "CNS", as used herein has its established meaning and refers to the brain and the spinal chord.

The present invention results from the surprising discovery that proteasomal degradation is responsible for the glucocorticoid insensitivity observed in patients suffering from ischemic disorders of the CNS; see the enclosed examples. It turns out that proteasomal degradation of the glucocorticoid receptor is specific for vascular endothelial cells. While glucocorticoid insensitivity in ischemic disorders of the CNS as such is an established fact, there is no remedy described in the art, the consequence being that the use of glucocorticoid is discouraged for these indications. The present invention makes glucocorticoid therapy available for such patients, wherein concomitant with such therapy glucocorticoid sensitivity is restored. Envisaged means for restoring glucocorticoid sensitivity are basically any means suitable to that effect, in particular the means according to items (ba) and (bb) of the main embodiment. The envisaged therapeutic approach is supported by the enclosed examples which include both *in vitro* and *in vivo* evidence. In particular, the capability of glucocorticoids to induce tight junction proteins at the blood brain barrier is restored upon interfering with the proteasomal degradation of the glucocorticoid receptor. While administration of only one of the constituents according to the combination of the main embodiment does not significantly ameliorate the symptoms of ischemic disorder, the combination does. Accordingly, the combinations according to the present invention are **characterized in that** they exhibit a synergistic effect; see the enclosed examples.

It is understood that by treating or preventing ischemic disorders of the central nervous systems, disorders which may arise in consequence of an ischemic disorder are also treated or prevented. Such disorders which may arise in consequence of an ischemic disorder include neurological disorders.

In a preferred embodiment, the glucocorticoid is selected from dexamethasone, methylprednisolone and cortisol. Another envisaged glucocorticoid is betamethasone. More preferred glucocorticoids are dexamethasone and methylprednisolone. Particularly preferred is methylprednisolone.

It established in the art, that different glucocorticoids, when administered as the sole pharmaceutically active agent, have different efficacy. For example, dexamethasone is known to be thirty times as potent as cortisol and approximately six times as potent as methylprednisolone. These known ratios between the potencies of the specific glucocorticoids have been taken into account when performing the experiments described in the examples enclosed herewith. In particular, dosages have been chosen which are described in the art as "equivalent dosages", i.e., dosages known to trigger comparable effects. Even though equivalent doses of the three GC were administered, efficacy surprisingly turned out to vary: the combination therapy with dexamethasone and bortezomib was most effective and consistent in reduction of stroke volume, edema volume and preservation of global neurological function. While the combination of methylprednisolone plus bortezomib had pronounced effects on edema volume, reduction of overall stroke volume und neurological damage was somewhat less effective as compared to dexamethasone. Both dexamethasone and methylprednisolone surprisingly performed superior when compared to cortisol. In other words, the preferred embodiments relating to the use of methylprednisolone and in particular of dexamethasone are characterized by a surprising advantageous technical effect which could not be predicted from the prior art.

In a further preferred embodiment, the proteasome inhibitor is selected from peptide boronic acids and esters thereof, lactacystin and analogs thereof, and peptide aldehydes. These compound classes are well known in the art as is their capability of inhibiting proteasome activity. They are inter alia described in WO 98/35691 A1 (cited above), which is incorporated by reference in its entirety. Particularly relevant is passage from page 11, bottom to page 12, line 22 of WO 98/35691 A1, which is reproduced in the following: Non-limiting examples of proteasome inhibitors for use in the present invention include peptide aldehydes (Stein et al. WO 95/24914 published September 21, 1995; Siman et al. WO 91/13904 published September 19, 1991; lqbal et al. J. Med. Chem. 38:2276-2277 (1995)), peptide boronic acids (Adams et al. WO 96/13266 published May 9, 1996; Siman et al. WO 91/13904), lactacystin, and lactacystin analogs (Fenteany et al. Proc. Natl. Acad. Sci. USA (1994) 91:3358; Fenteany et al. WO 96/32105, published 10/17/96). Peptide aldehyde proteasome inhibitors for use in the present invention preferably are those disclosed in Stein et al. WO 95/24914 published September 21, 1995 or Siman et al. WO 91/13904 published September 19, 1991, both hereby incorporated by reference in their entirety. Boronic acid or ester compounds for use in the present invention preferably are those disclosed in Adams et al. WO 96/13266 published May 9, 1996, or Siman et al. WO 91/13904, both of which are hereby incorporated by reference in their entirety. More preferably, the boronic acid compound for use in the present invention is selected from the group consisting of: N-(4-morpholine)carbonyl-β-(1-naphthyl)-L-alanine-L-leucine boronic acid; N-(8-quinoline)sulfonyl-β-(1-naphthyl)-L-alanine-L-alanine-L-leucine boronic acid; N-(2-pyrazine)carbonyl-L-phenylalanine-L-leucine boronic acid, and N-(4-morpholine)carbonyl-[O-(2-pyridylmethyl)]-L-tyrosine-L-leucine boronic acid. Lactacystin and lactacystin analog compounds for use in the present invention preferably are those disclosed in Fenteany et al. WO 96/32105, published 10/17/96, hereby incorporated by reference in its entirety. More preferably, the lactacystin analog is selected from lactacystin, *clasto*-lactacystin β-lactone, 7-ethyl-*clasto*-lactacystin β-lactone and 7-n-propyl-*clasto-*lactacystin β-lactone. Most preferably the lactacystin analog is 7-n-propyl-*clasto*-lactacystin β-lactone.

A preferred peptide aldehyde also known as calpain inhibitor I (N-acetyl-Leu-Leu-Nle-CHO) is one of the proteasome inhibitors used in the examples.

In a particularly preferred embodiment, the proteasome inhibitor is bortezomib. Bortezomib (systematic name: [(1S)-3-methyl-1-[[(2R)-3-phenyl-2-(pyrazine-2-carbonylamino)propanoyl]amino]butyl]boronic acid), also known as Velcade®, is a well-known proteasome inhibitor, which has been approved for the monotherapy of certain malignant diseases.

In particularly preferred embodiments of the present invention, the proteasome inhibitor is bortezomib and the glucocorticoid is selected from dexamethasone and methylprednisolone. Most preferred is the combination of bortezomib and dexamethasone. In these embodiments it is preferred that no further pharmaceutically active agent is comprised or used.

Further proteasome inhibitors according to the invention are RNAi agents, antisense molecules, ribozymes, antibodies and aptamers directed against one or more constituent proteins of the proteasome or nucleic acids encoding said constituent proteins, respectively.

The term "RNAi agent" refers to molecules capable of triggering RNA interference, RNA interference being a sequence-specific gene silencing process (see, e.g. Zamore Nat Struct Biol 2001, 8(9):746-50; Tuschl T. CHEMBIOCHEM. 2001, 2:239-245; Scherr and Eder, Cell Cycle. 2007 Feb;6(4):444-9; Leung and Whittaker, Pharmacol Ther. 2005 Aug;107(2):222-39; de Fougerolles et al., Nat. Rev. Drug Discov. 2007, 6: 443-453). "RNAi agents" include "small interfering RNAs" (siRNAs), which are a class of generally short and double-stranded RNA molecules that play a variety of roles in biology and, to an increasing extent, in treatment of a variety of diseases and conditions. Such siRNAs are generally 18-27 nt long, generally comprising a short (usually 19-21-nt) double-strand of RNA (dsRNA) with or without 2-nt 3' overhangs on either end. Each strand can have a 5' phosphate group and a 3' hydroxyl (-OH) group or the phosphate group can be absent on one or both strands. This structure is the result of processing by Dicer, an enzyme that converts either long dsRNAs or small hairpin RNAs into siRNAs.

RNAi agents can also be exogenously (artificially) introduced into cells by various transfection methods to bring about the specific knockdown of a gene of interest. In this context, other structures than those described above are also envisaged, provided they are capable of interfering with gene expression. Preferred are double-stranded RNAs (dsRNAs), the double-stranded part of which has a length of about 12 to about 50 base pairs. The term "dsRNA" includes siRNAs. The dsRNA of the invention may either have overhanging sequences of up to 10 bases, preferably not more than 5 bases in length at either end or at one end, or may be blunt-ended. Also preferred is that the complementarity to the target gene extends over the entire length of the double-stranded part. The region which is complementary to the target gene is at least 12 bases, preferably at least 15, 16, 17, 18, 19, 20, 21, 22, 23 or more bases in length. The siRNA of the invention may be fully complementary to the target gene. Alternatively, the dsRNA may comprise up to 5%, 10%, 20% or 30% mismatches to the target gene. Furthermore, dsRNAs (and also antisehse molecules) can be chemically modified e.g. on the backbone including the sugar residues. Preferred modifications of the dsRNA molecules of the invention include linkers connecting the two strands of the siRNA molecule. Chemical modifications serve *inter alia* to improve the pharmacological properties of siRNAs and antisense molecules such as *in vivo* stability and/or delivery to the target site within an organism. The skilled person is aware of such modified dsRNAs as well as of means and methods of obtaining them, see, for example, Zhang et al., Curr Top Med Chem. 2006;6(9):893-900; Manoharan, Curr Opin Chem Biol. 2004 Dec;8(6):570-9.

Essentially any gene of which the sequence is known can thus be targeted based on sequence complementarity with an appropriately tailored dsRNA. This has made dsRNAs an important tool for gene function and drug target validation studies as well as for therapeutic intervention which is envisaged here. The dsRNAs of the present invention are capable of reducing or blocking expression of the proteins and nucleic acids defined herein above.

The term "antisense molecules" or "antisense construct" is known in the art and refers to a nucleic acid which is complementary to a target nucleic acid. An antisense molecule according to the invention is capable of interacting with, more specifically hybridizing with the target nucleic acid. By formation of the hybrid, transcription of the target gene(s) and/or translation of the target mRNA is reduced or blocked. Standard methods relating to antisense technology have been described (see, e.g., Melani et al., Cancer Res. (1991) 51:2897-2901). Ribozymes are enzymes consisting of or comprising a catalytically active ribonucleic acid which in turn comprises a sequence complementary to a sequence in the target nucleic acid. Ribozymes specifically cleave the target nucleic acid, such as the (pre)-mRNA of a gene, the consequence being reduction or repression of expression. The techniques underlying said repression of expression are well known in the art (see, e.g., EP-B1 0 291 533, EP-A1 0 321 201, EP-A2 0 360 257). Selection of appropriate target sites and corresponding ribozymes can be done as described for example in Steinecke et al. (Methods in Cell Biology (1995) 50:449-460).

The term "antibody" includes monoclonal antibodies, polyclonal antibodies, single chain antibodies, or fragments thereof that specifically bind said peptide or polypeptide, also including bispecific antibodies, synthetic antibodies, antibody fragments, such as Fab, a F(ab₂)', Fv or scFv fragments etc., or a chemically modified derivative of any of these. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals with modifications developed by the art. Furthermore, antibodies or fragments thereof can be obtained by using methods which are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BIAcore system can be used to increase the efficiency of phage antibodies which bind to an epitope of the abovementioned protein (Schier, Human Antibodies Hybridomas 7 (1996), 97-105; Malmborg, J. Immunol. Methods 183 (1995), 7-13). The production of chimeric antibodies is described, for example, in WO89/09622. A further source of antibodies to be utilized in accordance with the present invention are so-catied xenogenic antibodies. The general principle for the production of xenogenic antibodies such as human antibodies in mice is described in, e.g., WO 91/10741, WO 94/02602, WO 96/34096 and WO 96/33735. Antibodies to be employed in accordance with the invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning: A Laboratory Manual, Cold Spring Harbor Laboratory, Cold Spring Harbor, NY, 1989. The term "monoclonal" or "polyclonal antibody" (see Harlow and Lane, (1988), loc. cit.) also relates to derivatives of said antibodies which retain or essentially retain their binding specificity. Whereas particularly preferred embodiments of said derivatives are specified further herein below, other preferred derivatives of such antibodies are chimeric antibodies comprising, for example, a mouse or rat variable region and a human constant region. The term "scFv fragment" (single-chain Fv fragment) is well understood in the art and preferred due to its small size and the possibility to recombinantly produce such fragments. Preferably, the antibody, aptamer (see further below), fragment or derivative thereof according to the invention specifically binds the target protein, (poly)peptide or fragment or epitope thereof whose presence or absence is to be monitored. The term "specifically binds" in connection with the antibody used in accordance with the present invention means that the antibody etc. does not or essentially does not cross-react with (poly)peptides of similar structures. Cross-reactivity of a panel of antibodies etc. under investigation may be tested, for example, by assessing binding of said panel of antibodies etc. under conventional conditions (see, e.g., Harlow and Lane, (1988), loc. cit.) to the protein of interest as well as to a number of more or less (structurally and/or functionally) closely related proteins. Only those antibodies that bind to the protein of interest but do not or do not essentially bind to any of the other proteins which are preferably expressed by the same tissue as the protein of interest, are considered specific for the protein of interest. In a particularly preferred embodiment of the method of the invention, said antibody or antibody binding portion is or is derived from a human antibody or a humanized antibody. The term "humanized antibody" means, in accordance with the present invention, an antibody of non-human origin, where at least one complementarity determining region (CDR) in the variable regions such as the CDR3 and preferably all 6 CDRs have been replaced by CDRs of an antibody of human origin having a desired specificity. Optionally, the non-human constant region(s) of the antibody has/have been replaced by (a) constant region(s) of a human antibody. Methods for the production of humanized antibodies are described in, e.g., EP-A1 0 239 400 and WO90/07861.

Aptamers are nucleic acid molecules that have been selected from random pools based on their ability to bind other molecules. Aptamers have been selected which bind nucleic acid, proteins, small organic compounds, and even entire organisms.

In a further preferred embodiment, the ischemic disorder of the central nervous system is a cerebral ischemic disorder or a spinal chord ischemia. Furthermore preferred is that the ischemic disorder of the central nervous system is selected from disruption of the blood brain barrier, cerebral infarct, cerebral stroke, brain edema, hypoxic conditions accompanying traumatic brain injury, ischemic conditions occurring in premature babies, brain hypoperfusion, embolic brain ischemia and thrombotic brain ischemia.
Ischemic disorders in general are **characterized in that** they entail glucose and or oxygen deprivation (hypoxia) of the affected tissue. In the central nervous system, both glucose deprivation and/or hypoxia may lead to leakiness of the blood brain barrier by a mechanism which has been investigated by the present inventors and is described in more detail in the examples. In brief, it is demostrated that occludin and claudin-5 are significantly reduced in murine brain-derived cEND cells upon hypoxia which leads to an increase in transendothelial permeability. Moreover, several other components of the junctional complex in brain endothelial cells such as claudin-3, -12 and VE-cadherin are likewise altered underlining that hypoxia has a critical impact on the structure of the neurovascular interface.

The term "blood brain barrier" is known in the art and refers to a separation of circulating blood and cerebrospinal fluid maintained by the choroid plexus in the central nervous system. Endothelial cells restrict the diffusion of microscopic objects (e.g. bacteria) and large or hydrophilic molecules into the cerebrospinal fluid, while allowing the diffusion of certain small molecules (such as O₂, hormones, CO₂). Cells of the barrier actively transport specific compounds such as glucose across the barrier with specific proteins.

The medical indications amenable to treatment according to the present invention include the ischemic conditions arising from stroke or infarct as defined above. The indications furthermore include ischemic/hypoxic conditions as they typically arise in prematurely born babies, in particular those with particularly low birth weight as well as in cases of traumatic brain injury. Prematurely born babies with particularly low birth weight include those with a birth weight below 1700 g.

In a preferred embodiment, the glucocorticoid is to be administered prior to, concomitantly with or after the proteasome inhibitor and/or the nucleic acid. It is understood that in those embodiments wherein the glucocorticoid is to be administered prior to or after the proteasome inhibitor, the time interval between the administration of the two agents is to be chosen such that the above described synergistic effect can occur. For example, at the point in time of the administration of a glucocorticoid following an earlier administration of a proteasome inhibitor, the amount of glucocorticoid receptor available for glucocorticoid signalling is elevated as compared to control subjects which have not been treated with a proteasome inhibitor.

Accordingly, it is preferred that the time interval between administration of the glucocorticoid and of the proteasome inhibitor and/or nucleic acid is not to exceed 48, 24, 12, 6, 3, or 0 hours. The time intervals are listed by increasing preference, i.e., 0 hours is the most preferred value.
The concomitant administration may be effected by administering a single dosage form which comprises both the glucocorticoid and the proteasome inhibitor and/or the nucleic acid. Alternatively, a single dosage form comprising the glucocorticoid and a separate single dosage form comprising the proteasome inhibitor and/or the nucleic acid may be administered concomitantly. Those embodiments where the glucocorticoid is to be administered prior to or after the proteasome inhibitor are typically effected by administering a single dosage form comprising the glucocorticoid and a single dosage form comprising the proteasome inhibitor and/or the nucleic acid at different points in time. In other words, the combination according to the invention implies one or more pharmaceutical compositions for use in the treatment of ischemic disorders of the central nervous system, the pharmaceutically active agents comprised in the pharmaceutical composition(s) being as defined above.

The recited administration may be performed once or repeatedly, dependent on the severity of the disorder and the needs of the patient. The patient may be a human or an animal. Preferred animals are mammals.

The pharmaceutical composition(s) will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of pharmaceutical composition administered to an individual will, inter alia, depend on the nature of the compound. For example, the total pharmaceutically effective amount of pharmaceutical composition administered parenterally per dose may be in the range of about 1 µg kg⁻¹ day⁻¹ to 10 mg kg⁻¹ day⁻¹ of patient body weight, although, as noted above, this may be subject to therapeutic discretion. More preferably, this dose is at least 0.01 mg kg⁻¹ day⁻¹, and most preferably for humans between about 0.01 and 1 mg kg⁻¹ day⁻¹. A preferred single or daily dose of dexamethasone for an adult is between 0.5 and 24 mg, more preferred between 0.5 and 8 mg, 0.5 and 4 mg and particularly preferred between 0.5 and 1.5 mg. A preferred single or daily dose of bortezomib is between 1 and 2 mg m⁻² body surface, 1.3 mg m⁻² body surface being a preferred value. If given continuously, the pharmaceutical composition is typically administered at a dose rate of about 1 µg kg⁻¹ hour⁻¹ to about 50 µg kg⁻¹ hour⁻¹, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

Pharmaceutical compositions of the invention may be administered orally, rectally, parenterally, intracisternally, intravaginally, intraperitoneally, topically (as by powders, ointments, drops or transdermal patch), bucally, or as an oral or nasal spray. The term "parenteral" as used herein refers to modes of administration which include intravenous, intramuscular, intraperitoneal, intrasternal, subcutaneous and intraarticular injection and infusion.

Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type.

The pharmaceutical composition is also suitably administered by sustained release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The solution for infusion or injection is prepared by reconstituting the lyophilized compound(s) using bacteriostatic water for injection.

Preferably, at least the glucocorticoid or at least the proteasome inhibitor and/or the nucleic acid is to be administered within 48, 24, 12, 6, 3, or 0 hours from the ischemic event. The time intervals are listed by increasing preference, i.e., 0 hours is the most preferred value. The term "ischemic event" refers to the point in time where the blockage or reduction in blood flow which is causative of the ischemic disorder occurred.

The present invention furthermore relates to at least one proteasome inhibitor and/or at least one nucleic acid encoding a glucocorticoid receptor, said glucocorticoid receptor being resistant to proteasomal degradation, for use in treating or preventing glucocorticoid resistance in ischemic disorders of the central nervous system. In this embodiment, the invention provides means for restoring glucocorticoid sensitivity in ischemic disorders of the central nervous system by counteracting proteasomal glucocorticoid receptor degradation and/or by providing a nucleic acid encoding a glucocorticoid receptor, said glucocorticoid receptor being resistant to proteasomal degradation. By restoring glucocorticoid sensitivity, the administration of glucocorticoids, otherwise not recommendable for ischemic disorders of the central nervous system, becomes a treatment option. In preferred embodiments, only one proteasome inhibitor and/or only one nucleic acid are used. More preferred is the use of one proteasome inhibitor or only one nucleic acid. Even more preferred is the use of one proteasome inhibitor.

Preferred embodiments of the proteasome inhibitor as well as of the recited ischemic disorder are defined herein above. A particularly preferred proteasome inhibitor is bortezomib.

In preferred embodiments, of the combination or the at least one nucleic acid according to the invention, said glucocorticoid receptor being resistant to proteasomal degradation is a glucocorticoid receptor wherein residues important for ubiquitin conjugation are mutated such that ubiquitin conjugation is abolished or substantially decreased. "Substantially decreased" includes less than 25%, less than 10%, less than 5%, less than 1%, less than 0,1 %, less than 0,01% or less than 0,001% ubiquitin conjunction as compared to the wild type GR, preferably from the same species. As described above, the proteasomal pathway involves the tagging of proteins for degradation. This tagging is effected by covalently linking an ubiquitin molecule to a molecule of the protein to be degraded, usually followed by covalently linking a further ubiquitin molecule to ubiquitin molecule attached to the protein to be degraded, and so on, thereby giving rise to polyubiquitin chain(s) attached to the protein to be degraded. Typical attachment sites on the target molecule for the first ubiquitin molecule are lysine residues. Mutating or deleting lysine residues is therefore a preferred option for rendering a protein less amenable to proteasomal degradation. Whether and to which extent a given mutation or deletion has the desired effect, can be determined by the skilled person without further ado, for example, by comparing the protein levels in cells transfected with the nucleic acid according to the invention as compared to untransfected controls. Determining protein levels can be performed with established methods such as Western blotting or immunoprecipitation.

It also envisaged to target other components of the proteasomal pathway, for example the enzymes involved in the process of ubiquitination (reviewed in Kinyamu (2005)), also known as ubiquitin ligases or E3 enzymes. The ubiquitin ligases generally are substrate-specific to a higher degree than the ubiquitin-conjugating enzymes (E2 enzymes) and the ubiquitin-activating enzymes (E1 enzymes) which are also part of the ubiquitination pathway, the ubiquitination pathway in turn being part of the proteasomal pathway according to the present invention. Accordingly, ubiquitin ligases, but also E1 and E2 enzymes are deliberately envisaged targets for interfering with proteasomal degradation of the glucocorticoid receptor. Inhibitors of E1, E2 and/or E3 enzymes are proteasome inhibitors in the sense of the above defined class (iv) of inhibitors.

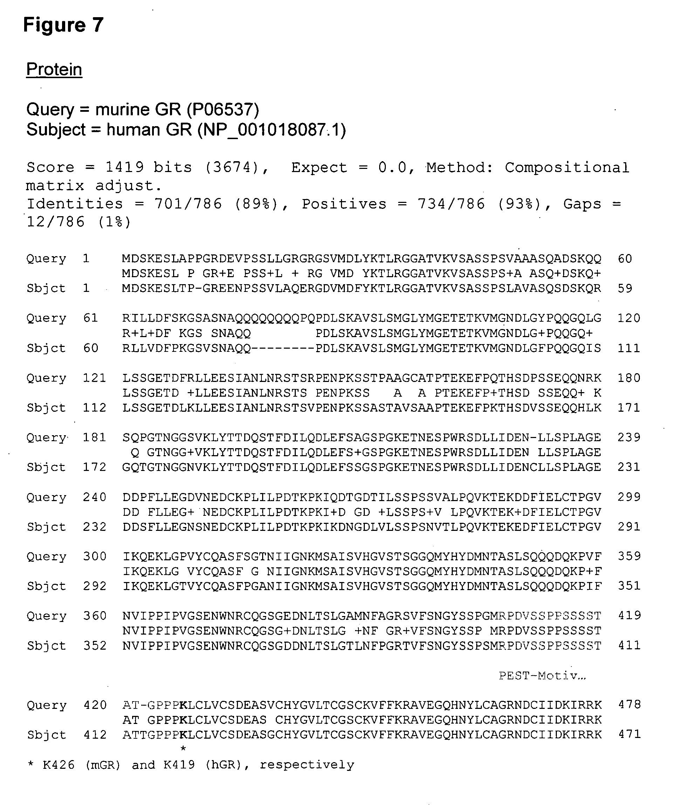

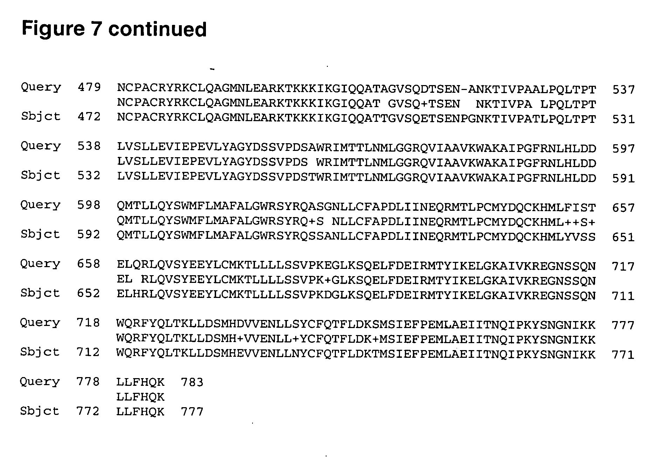

In preferred embodiments of the invention, said glucocorticoid receptor being resistant to proteasomal degradation is (i) a mutant of the human glucocorticoid receptor, the mutant differing from the wild type in that K419 is replaced with a different amino acid residue, or (ii) a mutant of a glucocorticoid receptor of a different mammalian species, said mutant of a glucocorticoid receptor of a different mammalian species differing from the corresponding wild type glucocorticoid receptor in that the lysine corresponding to position 419 of the human glucocorticoid receptor is replaced with a different amino acid residue. Preferably, lysine is replaced with alanine. It is shown in the examples that glucocorticoid receptor transactivation following glucocorticoid binding can be restored under ischemic conditions in cells transfected with the K426A mutant of the murine glucocorticoid receptor. Position 426 of the murine glucocorticoid receptor is the position corresponding to position 419 of the human glucocorticoid receptor. The sequences of SEQ ID NOs: 1 and 2 are those of the K419A mutant human glucocorticoid receptor (cDNA and amino acid sequence, respectively). The sequences of SEQ ID NOs: 3 and 4 are those of the K426A mutant murine glucocorticoid receptor (cDNA and amino acid sequence, respectively). The position of the lysine to be mutated in the glucocorticoid receptor of other species can be determined by the skilled person without further ado. In particular, the amino acid sequence of the glucocorticoid receptor of a given species may be aligned with the amino acid sequence of the human glucocorticoid receptor. The lysine aligning with the mutated position of the human sequence is the lysine to be mutated in the given species. Similarly, a multiple sequence alignment including human and/or murine glucocorticoid receptor sequences as well as sequences from one or more further species may be aligned. Algorithms for pairwise and multiple sequence alignment are known in the art. In brief, two nucleotide or protein sequences can be aligned electronically using suitable computer programs known in the art. Such programs comprise BLAST (Altschul et al. (1990), J. Mol. Biol. 215, 403-410), variants thereof such as WU-BLAST (Altschul & Gish (1996), Methods Enzymol. 266, 460-480), FASTA (Pearson & Lipman (1988), Proc. Natl. Acad. Sci. USA 85, 2444-2448) or implementations of the Smith-Waterman algorithm (SSEARCH, Smith & Waterman (1981), J. Mol. Biol. 147, 195-197). These programs, in addition to providing a pairwise sequence alignment, also report the sequence identity level (usually in percent identity) and the probability for the occurrence of the alignment by chance (P-value). Programs such as CLUSTALW (Higgins et al. (1994), Nucleic Acids Res. 22, 4673-4680) can be used to align more than two sequences. Preferably, default settings of the respective computer programs as published are used. A preferred alignment program is BLAST (*loc. cit*.). Figure 7 provides an alignment of the human and the murine glucocorticoid receptor amino acid sequences.

The present invention furthermore relates to a method of treating or preventing an ischemic disorder of the central nervous system, comprising the step of administering to a patient suffering from or an individual at risk of developing an ischemic disorder of the central nervous system (a) an effective amount of at least one glucocorticoid; and an effective amount (ba) of at least one proteasome inhibitor and/or (bb) of at least one nucleic acid encoding a glucocorticoid receptor, said glucocorticoid receptor being resistant to proteasomal degradation.

Also provided is a method of treating or preventing glucocorticoid resistance in ischemic disorders of the central nervous system, comprising the step of administering to a patient suffering from or an individual at risk of developing glucocorticoid resistance in an ischemic disorder of the central nervous system an effective amount of at least one proteasome inhibitor and/or of at least nucleic acid encoding a glucocorticoid receptor, said glucocorticoid receptor being resistant to proteasomal degradation.

Preferred embodiments of the invention, said preferred embodiments being defined herein above, also define preferred embodiments of the above methods of treatment.

The Figures show:

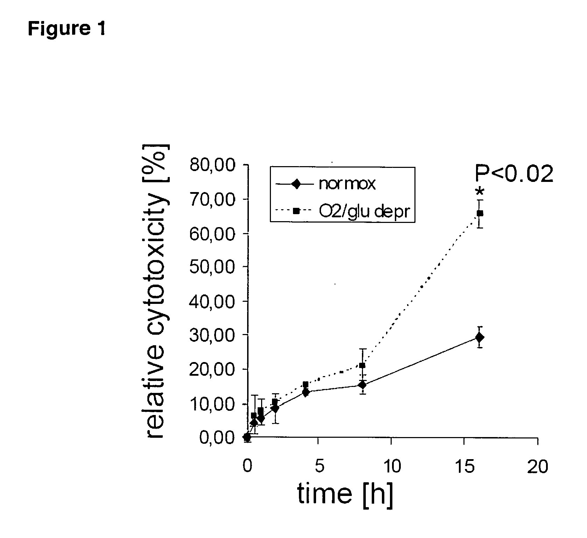

**Fig.1****. Establishment of an in vitro model for stroke-like conditions: O₂/glucose deprivation.**
   cEND cells were plated at a density of 1.1*10⁵ cells/3.5 cm² dish and grown to confluence. At confluence, cells were maintained at 2% FCS and O₂-deprivation or O₂/glucose-deprivation and glucocorticoids as indicated. After reaching confluence, cells were subjected for 2h, 4h, 8h and 16 h to O₂-deprivation in the presence and absence of glucose followed by a reperfusion of 24 h under normoxia with standard DMEM medium containg 11% glucose for all time points. Cell viability was by assessed by the release of LDH in response to O₂-deprivation or O₂/glucose deprivation: after the individual time points, the cytotoxicity and plasma membrane lysis was determined by analysis of release of intracellular LDH from damaged cells as compared to cells maintained under normoxic conditions. LDH analysis was performed by using the colorimetric LDH activity assay (Cytotoxicity Detection Kit (Roche)). Long term O₂/glucose deprivation increased plasma membrane damage significantly (P < 0.02) more than O₂ deprivation alone: after 2 h of O₂- or O₂/glucose-deprivation, cEND cells were damaged by 8.6 ± 3.6 % (O₂-depr and 10.66 ± 2.13 % (O₂/glu depr), after 4 h of O₂- or O₂/glucose-deprivation relative cytotoxicity was increased by 15.51 ± 1.1 % (O₂/glu depr) and 13.35 ± 0.9 % (O₂-depr), and after 16 h of incubation under O₂- or O₂/glucose-deprivation, a relative cytotoxicity of 66.1 ± 2.01 % (O₂/glu depr) and 29.62 ± 3.1 % (O₂-depr was determined.

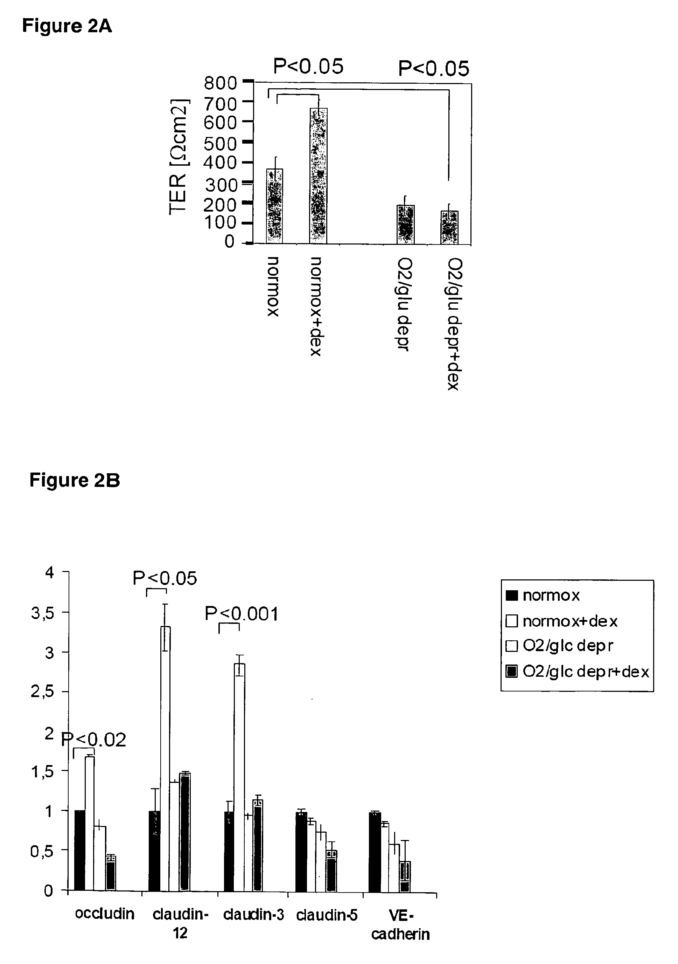

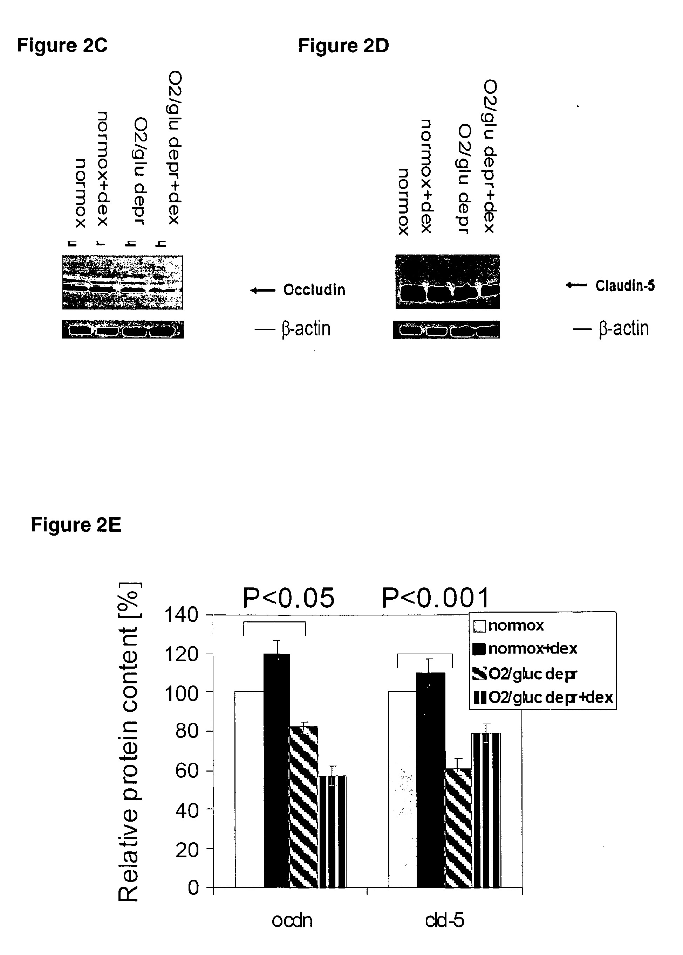

**Fig. 2****. Dexamethasone lacks barrier-tightening effects after O₂/glucose-deprivation.**
   **(A)** Glucocorticoid treatment does not restore compromised brain microvascular endothelial barrier function in response to O₂/glucose-deprivation: under normoxic conditions 24 h dexamethsone treatment (100 nM) significantly (P<0.05) increased TER values from 371 ± 59 Ωcm² (normox) to 671 ± 43 Ω cm² (normox+dex). After O₂/glucose-deprivation, measured TER of untreated cells was lowered to 195 ± 47 Ωcm² (O₂/glu depr). Dexamethasone administration during reoxygenation (O₂/glu depr+dex) did not increase TER values (167 ± 39 Ωcm²) as opposed to the observations under normoxic conditions.
   **(B)** Gene expression levels of genes encoding the BBB tight junction proteins occludin, claudins -3,-5,-12 and the adherens junction protein, VE-cadherin, were assessed by real time RT-PCR. We assessed gene induction by dexamethsaone under nomoxic conditions or after O₂/glucose deprivation. The validated GC target gene occludin was significantly (P<0.05) induced by dexamethasone under normoxic conditions. mRNA analyses revealed claudin-3 (P<0.001) and claudin-12 (P<0.05) as potential novel GR target genes in brain microvascular cEND cells. The genes for claudin-5 and VE-cadherin were on the contrary not induced by GC. After O₂/glucose deprivation, induction of genes encoding occludin, claudin-3 and claudin12 by dexamethasone was abolished pointing to GC insensitivity after O₂/glucose deprivation at the gene expression level. Normox control (black bars), normox+dex (white bars), O₂/gtu depr (light grey bars), O₂/glu depr +dex (dark grey bars).
   **(C)-(D)** The influence of O2/glucose-deprivation on the tight junction proteins occludin and claudin-5 could be shown by Western Blot: for this, cells were seeded on plastic cell culture flasks coated with collagen IV, treated as indicated and subjected to SDS-gel electrophoresis and Western blotting. A four-hour exposure to O₂/glucose-deprivation caused a significant (P<0.05) decrease in occludin protein to 82 ± 3 % as compared to the untreated control. While in cEND cells maintained in normoxia, dexamethasone administration elevated occludin protein contents to 120 ± 7 %, treatment with dexamethasone after O₂/glucose-deprivation did not recover occludin protein levels. After O₂/glucose-deprivation we even detected a further decrease of occludin protein by dexamethasone administration to 57 ± 5 %, as compared to cells maintained in normoxia. Comparably, O₂/glucose-deprivation caused a significant (P<0.001) decrease in claudin-5 protein to 61 ± 4 % as compared to the untreated control. Treatment with dexamethasone led to a slight increase in claudin-5 protein levels to 79 ± 5 %, as compared to untreated cells. Under normoxic conditions, dexamethasone administration did not significantly change claudin-5 contents as compared to untreated cells (110 ± 7 %) disclosing claudin-5 as a non-target for GC action.
   **(E)** Densitometric evaluation of **(c)** and **(d),** normox control (gray bars), normox+dex (black bars), O₂/glu depr (hatched bars), O₂/glu depr +dex (striped bars).

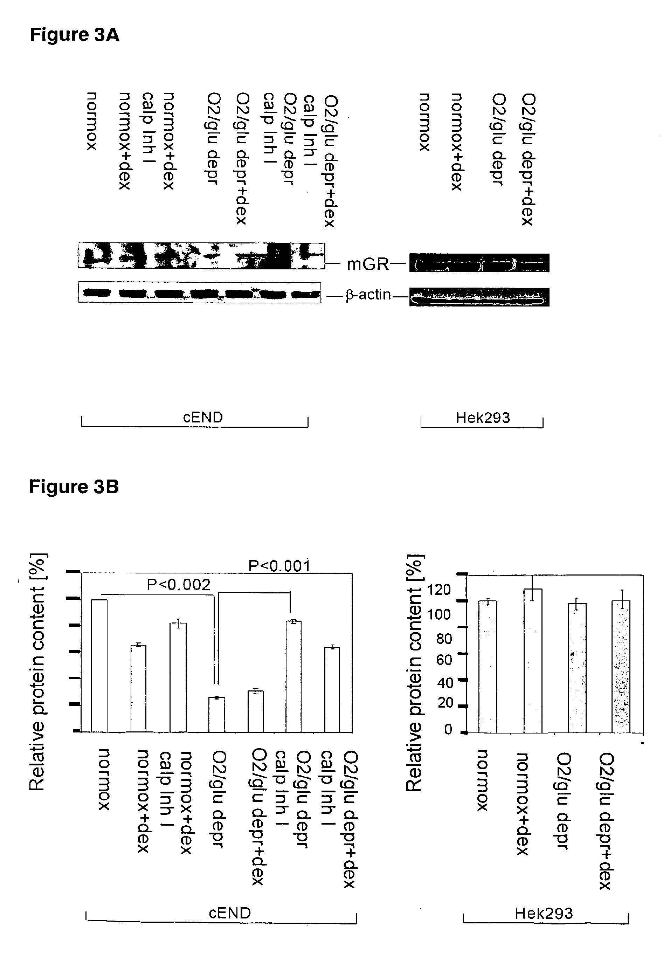

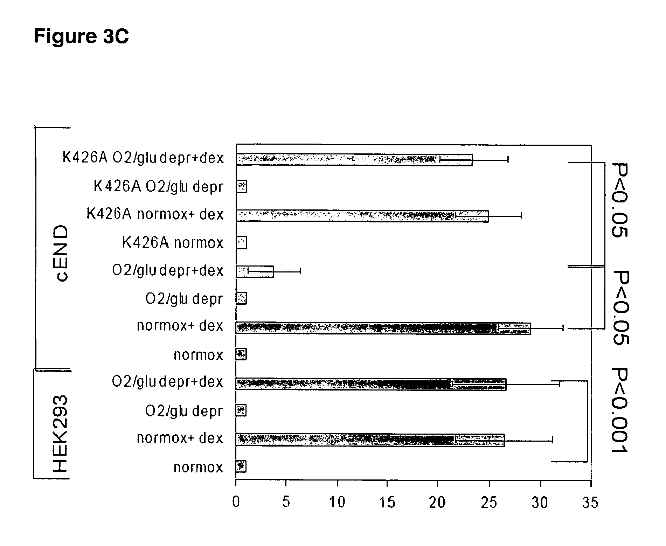

**Fig. 3****. O₂/glucose-deprivation causes GR insensitivity by proteasomal GR degradation**
   **(A)** GR protein levels after O₂/glucose-deprivation. Confluent monolayers of cEND cells were grown in collagen IV-coated cell culture flasks and exposed to normoxia or O₂/glucosedeprivation in the presence and absence of 100 nM dexamethasone and 25 µM calpain inhibitor I as indicated. In cell lysates from control cEND cells (normox), there was a strong signal for GR protein detectable. 24 h of dexamethasone treatment (normox+dex) led to a ligand-dependent reduction to 66 ± 2 % reduction in detectable GR protein (n = 3). In cEND cells exposed to O₂/glucose-deprivation (O₂/glu depr) GR protein was significantly (P<0.02) reduced to 26 ± 3 % of control (normox). 24 h of dexamethasone treatment during reoxygenation (O₂/glu depr +dex), slightly induced GR levels to 40 ± 4 % of control (normox) cells as detected by WB and densitometric analysis. In contrast, O₂/glucose deprivation in the presence of the proteasomal inhibitor calpain inhibitor I consistently and significantly (P<0.001) maintained GR protein levels at 84 ± 2 % of control (O₂/glu depr calp Inh I) as compared to cells not treated with calpain inhibitor I (O₂/glu depr). Additional dexamethasone treatment in the presence of calpain inhibitor I (O₂/glu depr, calp Inh I+dex) led to liganddependent reduction of GR protein levels to 64 ± 2 % of the untreated cEND cells under NG conditions.
   **(B)** GR transcriptional activation of a GC-responsive test promoter construct was monitored in normoxia and after O₂/glucose-deprivation. Subconfluent cEND and Hek293 cells were cotransfected with reporters pMMTV-luc (2) and pRL-TK (Promega), then grown to confluence and exposed to conditions and dexamethsone-treated as indicated.
   Cell extracts were assayed for Luc and Renilla activity. The transactivation activity without dexamethasone was considered as the baseline activity of the promoter.
   While in Hek293 cells, MMTV promoter is transactivated by dexamethasone 26.45 ± 4.8-fold in normoxia and 26.6. ± 5.3-fold after O₂/glucose-deprivation (P<0.001) (n = 4) by endogenous GR, in cEND cells, significantly reduced (P<0.05) transactivation by GR (3.7 ± 2.6-fold) in endothelial cEND cells after O₂/glucose-deprivation as opposed to transactivation in cEND cells in normoxia (29.01 ± 3.2-fold) (normox+dex) was observed. Co-transfection of cEND cells with the mutant GR-K426A resistant to proteasomal degradation resulted in significantly (P<0.05) restored transactivation of the MMTV test promoter after O₂/glucosedeprivation, (23.4 ± 3.3-fold), which is comparable to GR transactivation activity of K426A-transfected cEND cells in normoxia (24.9 ± 3.2-fold). The results are displayed as the ratio of activity with ligands to activity without (mean and s.d. from at least 4 experiments).

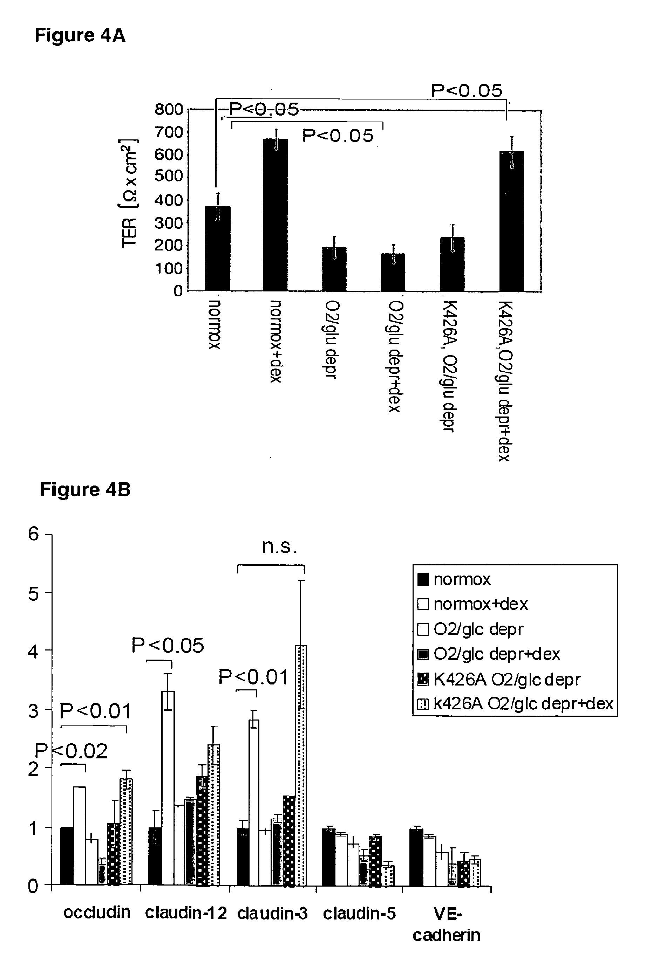

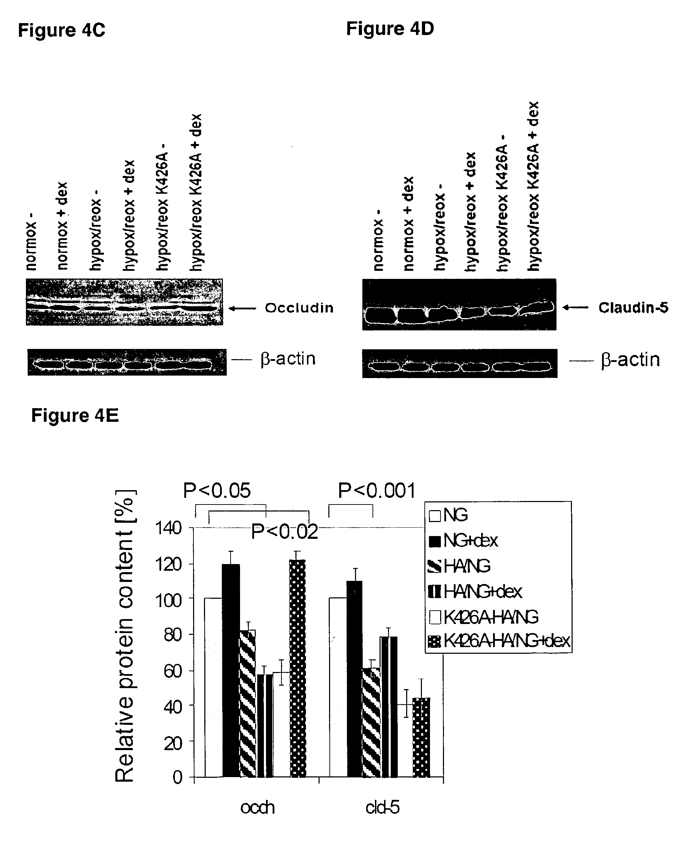

**Fig. 4****. Proteasomal inhibition restores barrier-tightening effects of dexamethasone after O₂/glucose-deprivation.**
   **(A)** Effects of dexamethasone on TER of cEND monolayers were detected in cEND cells transfected with the mutant GR-K426A defective for proteasomal degradation. Growth medium (10 % FCS) was changed after 5 days in culture to differentiation medium (2 % FCS, ± additions) and analysis of the TER was performed after O₂/glucose-deprivation as indicated. Treatment with 100 nM dex leads to an increase in TER to 671+43 Ωcm² (normox+dex)(P<0.05) as compared to the untreated control (normox) (371+59 Ωcm²). After O₂/glucose-deprivation, TER was reduced to 195+47 Ωcm² (O₂/glu depr) (P<0.05), and simultaneous treatment with dexamethasone (O₂/glu depr +dex) did not lead to elevated resistances (166+39 Ωcm²). For GR- K426A-transfected cEND cells without dexamethasone administration (K426A O₂/glu depr), O₂/glucose-deprivation equally led to reduced TER (239+58 Ωcm²). As expected, treatment of K426A-transfected cells with dexamethasone (K426A O₂/glu depr +dex) conditions led to full GC response as demonstrated by a significantly (P<0.05) increased TER (619±67 Ωcm²).
   **(B)** At the mRNA level, transcripts of the glucocorticoid-responsive genes (occludin, claudin-12 and claudin-3) were significantly (P<0.01, occludin) upregulated in dexamethasone-treated cEND cells overexpressing the mutant K426A after O₂/glucose-deprivation. Normox control (black bars), normox+dex (white bars), O₂/glu depr (light grey bars), O₂/glu depr +dex (dark grey bars), K426A, O₂/glu depr (light dotted bars), K426A, O₂/glu depr+dex (dark dotted bars)
   **(C)** Effects of proteasomal inhibition on occludin protein levels were examined by western blot analysis and densitometric analysis. For this, GR-426A-transfected cEND cells were seeded on plastic cell culture flasks coated with collagen IV und subjected to O₂/glucosedeprivation and treated with dexamethasone as indicated.
      Mirroring observations at the mRNA level, O₂/glucose-deprivation caused a decrease in occludin protein to 82 ± 3 % as compared to the untreated control and treatment with dexamethasone did not recover occludin loss, we detected a further decrease to 57 ± 5 % (P<0.05), as compared to cells in normoxia (normox). Introduction of the mutant GR-K426A led to an elevated significant (P<0.02) occludin protein synthesis when dexamethasonetreated after O₂/glucose-deprivation (122 ± 5 %)(K426A O₂/glu depr +dex) which was not observed without dexamethasone administration in cells after O₂/glucose-deprivation (K426A O₂/glu depr)(58 ± 7 %).
   **(D)** Effects of proteasomal inhibition on claudin-5 protein levels O₂/glucose-deprivation in the presence (44 ± 11 %)(K426A O₂/glu depr +dex) and absence (41 ± 8 %)(K426A O₂/glu depr) of dexamethasone in GR-K426A-transfected cEND cells led to a severe reduction in claudin-5 protein levels, which were even stronger than in the untransfected cEND cells after O₂/glucose-deprivation (61 ± 4 %).
   (E) Densitometric evaluation of (C) and (D), NG control (gray bars), NG+dex (black bars), HA/NG (hatched bars), HA/NG+dex (striped bars), K426A GR HA/NG (white bars), K426A GR HA/NG+dex (dotted bars)

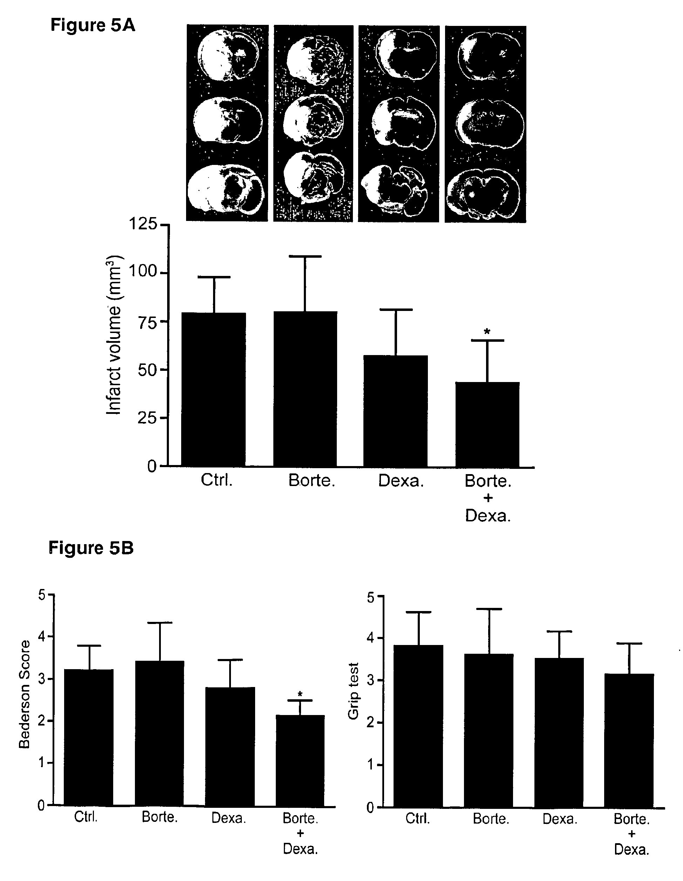

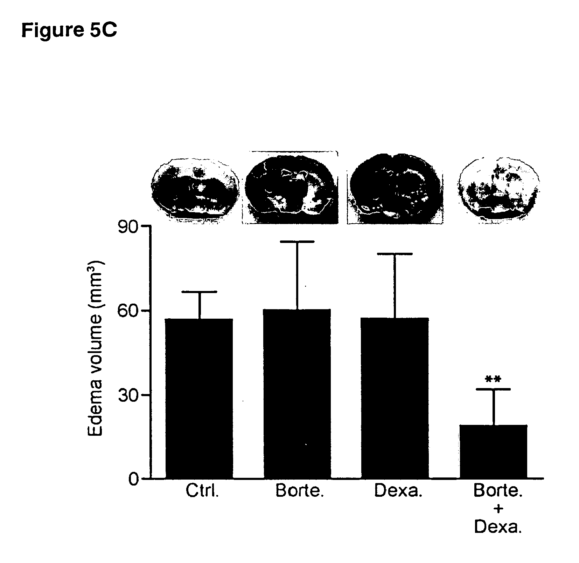

**Fig. 5****. Infarct volumes, functional outcomes and brain edema formation 24 hours after tMCAO in untreated and dexamethasone+bortezomib-treated mice.**
   **A)** Representative TTC stains (upper panel) of 3 correspondig coronal brain sections of mice untreated (Ctrl.), bortezomib-treated mice (Borte.), dexamethasone-treated mice (Dexa.) and mice treated with the combination of dexamethasone and bortezomib (Borte.+Dexa.). Brain infarct volumes (lower panel) as measured by planimetry in mice untreated (Ctrl.), bortezomib-treated mice (Borte.), dexamethasone-treated mice (Dexa.) and mice treated with the combination of dexamethasone and bortezomib (Borte.+Dexa.).
   **B)** Neurological Bederson score (left panel) and grip test score (right panel) of mice (n=10/group) at day 1 after tMCAO in mice untreated (Ctrl.), bortezomib-treated mice (Borte.), dexamethasone-treated mice (Dexa.) and mice treated with the combination of dexamethasone and bortezomib (Borte.+Dexa.).
   **C.** Representative corresponding coronal brain sections (upper panel) of mice untreated (Ctrl.), and mice untreated (Ctrl.), bortezomib-treated mice (Borte.), dexamethasone-treated mice (Dexa.) and mice treated with the combination of dexamethasone and bortezomib (Borte.+Dexa.) at day 1 after tMCAO after injection of Evans's blue. Note the the strongly reduced edema volume in mice treated with the combination of dexamethasone and bortezomib (Borte.+Dexa.). Volume of Evans's blue extravasation (lower panel) determined by planimetry in the ischemic hemisphere of untreated untreated mice and mice treated with the combination of dexamethasone and bortezomib 24 hours after 60 minutes of tMCAO (n=4/group).

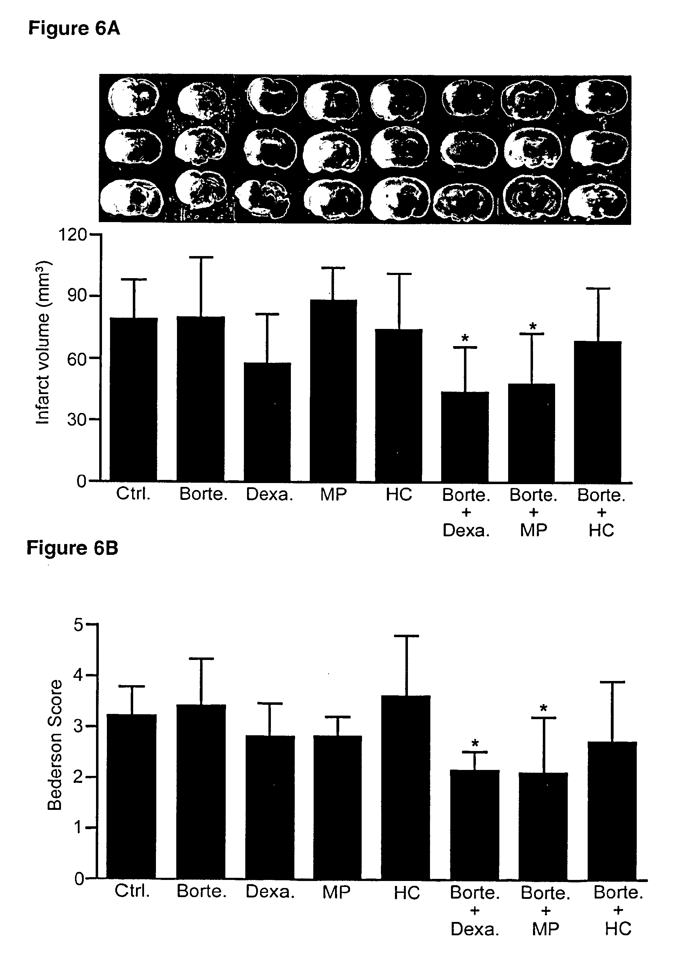

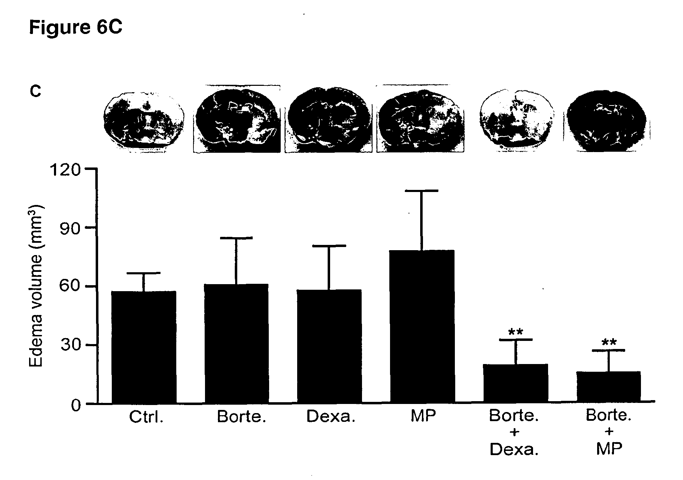

**Fig. 6****. Comparison of infarct volumes, functional outcomes and brain edema formation 24 hours after tMCAO in untreated mice and mice treated with different GC +bortezomib combinations.**
   **A)** Representative TTC stains (upper panel) of 3 corresponding coronal brain sections of mice untreated (Ctrl.), bortezomib-treated mice (Borte.), dexamethasone-treated mice (Dexa.), methylprednisolone-treated mice (MP), cortisol-treated mice (HC) and mice treated with the combinations dexamethasone and bortezomib (Borte.+Dexa.), methylprednisolone and bortezomib (Borte+MP) and cortisol and bortezomib (Borte+HC). Brain infarct volumes (lower panel) as measured by planimetry in mice untreated (Ctrl.), bortezomib-treated mice (Borte.), dexamethasone-treated mice (Dexa.) and mice treated with the combination of dexamethasone and bortezomib (Borte.+Dexa.).
   **B)** Neurological Bederson score of mice (n=10/group) at day 1 after tMCAO in mice untreated (Ctrl.), bortezomib-treated mice (Borte.), dexamethasone-treated mice (Dexa.), methylprednisolone-treated mice (MP), cortisol-treated mice (HC) and mice treated with the combinations dexamethasone and bortezomib (Borte.+Dexa.), methylprednisolone and bortezomib (Borte+MP) and cortisol and bortezomib (Borte+HC).
   **C.** Representative corresponding coronal brain sections (upper panel) of mice untreated (Ctrl.), bortezomib-treated mice (Borte.), dexamethasone-treated mice (Dexa.), methylprednisolonetreated mice (MP), cortisol-treated mice (HC) and mice treated with the combinations dexamethasone and bortezomib (Borte.+Dexa.), methylprednisolone and bortezomib (Borte+MP) and cortisol and bortezomib (Borte+HC) at day 1 after tMCAO after injection of Evans's blue. Note the strongly reduced edema volume in mice treated with the combination of dexamethasone and bortezomib (Borte.+Dexa.). Volume of Evans's blue extravasation (lower panel) determined by planimetry in the ischemic hemisphere of untreated untreated mice and mice treated with the combination of dexamethasone and bortezomib 24 hours after 60 minutes of tMCAO (n=4/group).
**Fig. 7****. Alignment of human and murine glucocorticoid receptor sequences.**
The database identifiers refer to the NCBI protein sequence database status of September 18, 2009.

The following examples illustrate the invention but should not be construed as being limiting.

### Example 1

### Methods

**Inhibitors and drugs.** Dexamethasone, methylprednisolone and cortisol and calpain inhibitor I (LLnL, N-acetyl-Leu-Leu-Nle-CHO) were purchased from Sigma, Taufkirchen, Germany.

### Animal experiments

Animal experiments were approved by the Regierung von Unterfranken and conducted according to the recently published recommendations for research in mechanism-driven basic stroke studies (Dirnagl (2006)).

*Induction of focal cerebral ischemia.* Focal cerebral ischemia was induced in 6-8-weeks old mice by 60 min transient middle cerebral artery occlusion (tMCAO) as described (Kleinschnitz (2006)). Briefly, mice were anesthetized with 2.5% isoflurane (Abbott, Wiesbaden, Germany) in a 70% N2O/30% O₂ mixture. Following a midline skin incision in the neck, the proximal common carotid artery and the external carotid artery were ligated and a standardized silicon rubber-coated 6.0 nylon monofilament (6021; Doccol Corp., Redlands, CA, USA) was inserted and advanced via the right internal carotid artery to occlude the origin of the right MCA. Operators (TS, CK) were blinded to the treatment groups and operation time per animal did not exceed 15 minutes. The intraluminal suture was left in situ for 60 minutes. Then animals were re-anesthetized and the occluding monofilament was withdrawn to allow reperfusion. 2 h after reperfusion mice were treated with intraveneous injections of either dexamethasone (Sigma-Aldrich, Taufkirchen, Germany; 10 mg/kg), bortezomib (Janssen-Cilag GmbH, Neuss, Germany, 0.2 mg/kg) or the combination of both substances (n=10/group). Control mice (n=10) received 0.9% NaCl solution. Assessment of functional outcome, determination of infarct size and determination of brain edema volume were carried following standard procedures (Austinat (2009)).

For comparison of the efficacy of a combination therapy with bortezomib with either of two commonly used steroids in the clinical practise (dexamethasone and methylprednisolone, respectively) and of the endogenous GC cortisol, mice were treated with equivalent doses of the GC: dexamethasone (Sigma-Aldrich, Taufkirchen, Germany; 10 mg/kg), methylprednisolone (Sigma-Aldrich, Taufkirchen, Germany; 60 mg/kg) and cortisol (Sigma-Aldrich, Taufkirchen, Germany; 300 mg/kg) and bortezomib (Janssen-Cilag GmbH, Neuss, Germany, 0.2 mg/kg) or the combination of either GC with bortezomib (n=10/group). Control mice (n=10) received 0.9% NaCl solution. Assessment of functional outcome, determination of infarct size and determination of brain edema volume were carried following standard procedures (Austinat (2009)).

**Isolation and culture of cerebral endothelial cells.** The immortalized mouse brain capillary endothelial cell line cEND was generated as described (Förster (2005)).

**Cell cultures, in vitro-stroke conditions and cell viability studies. cEND** cells were cultivated as described (Förster (2008)). Hek293 cells were maintained in DMEM, 10 % FCS. For normoxic conditions (normox) cells were maintained in an atmosphere of 5.0% CO₂ / 95 % O₂ and at 37 °C in DMEM, 2 % FCS . For the establishment of O₂/glucose deprivation conditions (O₂/glu depr), cells were maintained in an atmosphere of 1% O₂ balanced with N2 in glucose-free DMEM, 2% FCS for 4 h and reoxigenated in DMEM medium, containing 11 % glucose, 2 % FCS in an atmosphere of 5.0% CO₂ / 95 % O₂ at 37 °C for 24 h for all experiments. We performed cell viability assays following standard procedures.

### Electrophoresis and immunoblotting; Quantitative real-time RT-PCR, Transendothelial electrical resistance measurements, Transfection and luciferase assay.

Electrophoresis and immunoblotting, quantitative real-time RT-PCR, transendothelial electrical resistance measurements, transfection and luciferase assay were performed following standard procedures.

**Analysis and Statistics.** Values for densitometry, gene expression and promoter transactivation were averaged to establish a single value for control cells and treated cells as indicated. Values were compared through analysis of variance (ANOVA) with Bonferroni post hoc test: P values < 0.05 were considered significant (*).

### Example 2

### Establishment of an in vitro model for ischemic/hypoxic conditions

The brain endothelium becomes rapidly activated during ischemic stroke and structural BBB damage can already be found several hours after stroke onset in rodents and humans (del Zoppo (2001), Rosenberg (2009)). GC, like the therapeutically administered dexamethasone, have been demonstrated to stabilize BBB integrity in many diseases like Multiple Sclerosis or in the adjuvant treatment of brain tumours, mainly by the induction of tight and adherens junction proteins as direct or indirect target genes (Förster (2007), Romero (2003), Blecharz (2008), Koehler (1995)). However, barrier tightening or preserving effects could not be observed in the treatment of stroke patients (3). To resolve the molecular basis for this obvious GC insensitivity or resistance under conditions of O₂/glucose deprivation, we established in vitro conditions for O₂/glucose deprivation utilizing our cEND in vitro model of the BBB (Forster (2005)). For the establishment of standardized stroke-like conditions, we first compared the extent of cellular damage by stroke-like conditions (O₂/glucose deprivation) to the effects of exposure to O₂-deprivation alone **(****Fig. 1****).** Measuring cellular lactate dehydrogenase (LDH) release reflecting plasma membrane damage (Kim (2009)), our analysis revealed that additional glucose deprivation aggravates the deleterious effects of O₂ deprivation alone on brain microvascular endothelial cells over time **(****Fig. 1****).** While the extent of cytotoxicity was comparable between the two experimental setups, long term O₂/glucose deprivation increased plasma membrane damage significantly (P < 0.02) more, 66.1 ± 2.01 % (O₂/glu depr), than O₂ deprivation alone, 29.62 ± 3.1 % (O₂ depr) **(****Fig. 1****.).** The following experiments were carried out to analyse the effects of GC on brain endothelial barrier function during reoxigenation after in vitro strokelike conditions (O₂/glucose deprivation). Specifically, cEND cells were exposed to 4 h of O₂/glucose deprivation followed by reoxygenation for 24 h in the presence or absence of dexamethasone as a standardized in vitro model.

### Dexamethasone has no barrier protecting effects on brain microvascular endothelial cells in in vitro-stroke conditions.

Analysis of GC effects on brain endothelial barrier integrity under normoxic and O₂/glucose-deprived conditions revealed strong differences in GC efficacy. Exposure of cEND monolayers to O₂/glucose deprivation compromised barrier tightness as reflected by a significant (P<0.05) decrease in TER, 195 ± 47 Ωcm² (O₂/glu depr), as compared to control monolayers maintained under normoxic conditions, 371 ± 59 Ωcm² (normox) **(****Fig. 2A****).** Additional GC administration led to a significant (P<0.05) increase in monolayer tightness under normoxic conditions, 671 ± 43 Ωcm² (normox +dex) **(****Fig. 2A****),** but administration of dexamethasone to cEND monolayers during reoxigenation after O₂/glucose deprivation could not restore compromised BBB integrity in vitro, 167 ± 39 Ωcm² **(****Fig. 2A****).** These results parallel the outcome of dexamethasone therapy in clinical experience (Poungvarin (2004)). Our data thus pointed to reduced cellular sensitivity of brain microvascular endothelial cells to GCs in vitro following O₂/glucose deprivation, and validated our in vitro model for the further analysis of the molecular background of GC insensitivity after 02/glucose deprivation.

### O₂/glucose deprivation leads to reduced expression of tight junction encoding genes

Alterations in barrier tightness are mainly reflected by the expression levels of tight junction proteins sealing the paracellular space (Forster (2008)). We compared gene expression levels between monolayers maintained under nomoxic conditions or after O₂/glucose deprivation and after dexamethasone administration **(****Fig. 2B****).** Moreover, gene induction by dexamethasone was compared under both conditions to reveal potential differences in GC-mediated gene expression control after O₂/glucose deprivation.

At first, expression levels of genes encoding the tight junction proteins occludin, claudins -3, -5, -12 and the adherens junction protein, VE-cadherin, were assessed by real time RT-PCR **(****Fig. 2B****).** We compared gene expression levels between monolayers maintained under nomoxic conditions or after O₂/glucose deprivation and after dexamethasone administration **(****Fig. 2B****).** For the validated GC target gene occludin (Harke (2008), Forster (2008)), real-time RT-PCR analysis confirmed significant (P<0.02) induction (1.7 ± 0.1-fold) by dexamethasone under normoxic conditions as previously described (Forster (2005)). mRNA analyses of cEND monolayers treated with dexamethasone revealed moreover claudin-3, 2.85 ± 0.14-fold induction, (P<0.001) and claudin-12, 3.3 ± 0.3-fold induction, (P<0.05) as potential novel GR target genes at the BBB **(****Fig. 2B****).** On the contrary, genes for claudin-5 and VE-cadherin did not show GC induction, thereby excluding them from the potential candidate GC target genes at the BBB **(****Fig. 2B****).** After O₂/glucose deprivation, induction of the identified GC target genes, occludin, claudin-3, claudin-12, by the GC dexamethasone was not detected. For all these genes showing GC-responsivity in normoxia, dexamethasone administration failed to increase transcript levels after O₂/glucose deprivation proving GC insensitivity following O₂/glucose deprivation at the gene expression level **(****Fig. 2B****).** Expression levels of non-targets of GR action, VE-cadherin and claudin-5, were even diminished.

### O₂/glucose deprivation disrupts brain endothelial tight junction proteins

The tight junction proteins occludin and claudin-5 are described to be the key mediators of BBB sealing and maintenance (D'Atri (2002)). A measurable reduction in tight junction protein levels and lack in GC effects would establish the morphological correlate for the increased monolayer permeability detected. Therefore, we next assessed the effects of O₂/glucose deprivation in the presence and absence of dexamethasone on occludin and claudin-5 protein levels **(****Fig. 2C-E****).** As expected, exposure of cEND monolayers to O₂/glucose deprivation led to significant reduction in measurable tight junction protein levels for occludin to 82 ± 3 % (P<0.05) and for claudin-5 to 61 ± 4 % (P<0.001) of control levels. While for the validated GC-target occludin, levels were elevated to 120 ± 7 % of control by dexamethasone under normoxic conditions, no increase in levels of this major barrier sealing protein was detected by western blot analysis after O₂/glucose deprivation.

Dexamethasone failed to induce an increase in levels of the GC target protein occludin after O₂/glucose deprivation. GC effects at the protein level thus mirrored observations at the gene expression level, indicating that effects at the gene expression level are chiefly responsible for variances in barrier restoration by GC.

As expected, claudin-5 induction by dexamethasone was not observed under either condition ruling out claudin-5 as a directly regulated target of GC at the BBB **(****Fig. 2D-E****).** As observed at the mRNA level, claudin-5 protein levels were down-regulated following dexamethasone treatment under conditions of O₂/glucose deprivation.

### GC insensitivity in hypoxia is reversed by inhibition of proteasomal GR degradation

In an effort to explain the lack of GC-sensitivity and -response of cEND cells exposed to O₂/glucose deprivation, we compared GR protein levels in cEND cells maintained in the presence and absence of dexamethasone in normoxic conditions or after O₂/glucose deprivation. We further determined GR activity by promoter-reporter gene assay **(****Fig. 3****).**

As expected for GC responsive cells (Zhou (2005)), GR protein was readily detectable by western blot analysis in cEND cells under normoxic conditions. In response to ligand (dexamethasone) exposure, GR protein levels were down-regulated to 66±2 % of untreated cells as previously described **(****Fig. 3A,B****)**(Forster (2006)). Strikingly, in cEND cells exposed to O₂/glucose deprivation, significantly (P<0.002) reduced cellular GR protein levels were detected even in absence of ligand **(****Fig. 3A,B****).** GR protein amounts were reduced to the detection limit (26±3 % of control) and were not significantly influenced by dexamethasone administration during reoxigenation **(****Fig. 3A,B****).** In contrast, in the renal tubular epithelial cell line Hek293 which has been described to maintain GC sensitivity after O₂/glucose deprivation (Leonard (2005)), GR protein levels were not reduced after O₂/glucose deprivation, whether in the presence or the absence of dexamethasone **(****Fig. 3A,B****).** Since the extent of GC sensitivity is linked to cellular GR expression levels in a given cell type (Zhou (2005)), these findings suggested an explanation for the hormone insensitivity of cEND cells after O₂/glucose deprivation **(****Fig. 3A,B****).** In an effort to restore cellular GC sensitivity of cEND cells, we aimed to counteract proteasomal GR degradation during O₂/glucose deprivation by addition of the proteasome inhibitor calpain inhibitor I (A6185) (Groll (2004), Vinitsky (1994)) to the incubation media. Proteasomal inhibition prevented reduction of GR protein levels in cEND cells in response to O₂/glucose deprivation as compared to cells exposed to O₂/glucose deprivation in the absence of calpain inhibitor I (P<0.001) **(****Fig. 3A,B****).** Since the extent of GC sensitivity is linked to cellular GR expression levels in a given cell type (Zhou (2005)), these findings suggested an explanation for the hormone insensitivity of cEND cells after O₂/glucose deprivation **(****Fig. 3a,b****).** Our results unambiguously demonstrate that excessive proteasomal degradation of the GR is chiefly responsible for alterations in GR protein levels in brain microvascular cEND cells after O₂/ glucose deprivation. Further, this appears to be a specific feature of the vascular endothelium, since epithelial Hek293 cells did not show GR downregulation in response to O₂/glucose deprivation.

### Proteasomal inhibition restores GR transactivation of target genes

*In vitro* data:

The major biological function of steroid hormone receptors like GR is control of gene expression. To test whether the synergistic effect of proteasomal inhibition and dexamethasone administration would restore GR activity, we tested GR-mediated transactivation of a well described GC-responsive test promoter, MMTV (Benore-Parsons (1991)). The MMTV test promoter which contains five classical consensus GREs (5'-GTTACAnnnTGTTCT-3') (Benore-Parsons (1991)) was introduced into cEND and HEK293 cells under the different experimental conditions. MMTV promoter transactivation under normoxic conditions in dexamethasone-treated cEND cells (29.01 ± 3.2-fold) and Hek293 cells (26.45 + 4.8-fold), respectively, compared well to the literature **(****Fig. 3c****).** However, while in Hek293 cells MMTV promoter transactivation was still significant (26.6. ± 5.3-fold)(P<0.001) after O₂/glucose deprivation, in endothelial cEND cells MMTV transactivation was significantly (P<0.05) diminished (3.7 ± 2.6-fold) mirroring the reduction in GR protein levels detected under these conditions **(****Fig. 3C****).**

We were able to reverse this loss of receptor activity by introduction of the mutant GR-K426A into cEND cells. The mutant GR-K426A is resistant to proteasomal targeting due to a point mutation at the position lysine-426 important for ubiquitin conjugation to the GR protein destined for degradation (Wallace (2001)). Under both normoxic and hypoxic conditions, cEND cells overexpressing the mutant GR-K426A showed comparable MMTV test promoter transactivation levels **(****Fig. 3c****).** After O₂/glucose deprivation, MMTV promoter transactivation was significantly (P<0.05) higher in cells cotransfected with the mutant GR-K426A than in untransfected cEND cells. In fact, cEND cells overexpressing the mutant GR-K426A showed equal GC responsivity, under both normoxic. (24.9 ± 3.2-fold) and hypoxic (23.4 ± 3.3-fold) conditions, unambigously proving dysregulated proteasomal degradation of GR under O₂/glucose deprivation caused the GC insensitivity.

Analysis of the combined effects of GC administration and proteasome inhibition following O₂/glucose deprivation was expanded to BBB integrity. Functional analyses included measurement of monolayer tightness (TER) and gene and protein expression analyses of cEND cells transfected with the GR-K426A mutant under the different experimental conditions. While O₂/glucose deprivation led to a measurable decrease of TER in GR-K426A-transfected cells (239+58 Ωcm2)(K426A O₂/ glu depr), we detected a significant (P<0.05) positive increase in monolayer tightness in GR-K426A-transfected cells in response to dexamethasone administration following O₂/glucose deprivation (619±67 Ωcm2), as observed for dexamethasone-treated cEND cells in normoxia **(****Fig. 4A****).**

The glucocorticoid-responsive genes (occludin, 1.82 ± 0.15-fold, claudin-12, 2.4 ± 0.3-fold, and claudin-3, 4.1 ± 1.1-fold) (P<0.01, occludin) were strongly upregulated in GR-K426A-transfected cells exposed to O₂/glucose deprivation to a magnitude comparable to levels detected in cEND cells maintained in normoxia (Fig. 4B). Likewise, at the protein level, significantly (P<0.02) increased levels of the GC-target occludin by dexamethasone to 122 ± 5 % of control could be detected following proteasomal blockade
during O₂/glucose deprivation, most probably contributing to the positive increase in monolayer tightness determined **(****Fig. 4C,E****).**

Non-GR targets (claudin-5, VE-cadherin) were not influenced at the gene expression level **(****Fig 4B****)** or at the protein level **(****Fig. 4B****,D,E)** by the introduction of GR-K426A, underlining the divergent effects of GC/GR action on different barrier constituting proteins in brain microvascular endothelium.

*In vivo* data.

We subsequently hypothesized that the dual approach of GC treatment under proteasomal inhibition might also be beneficial under hypoxia in vivo, e.g. following acute ischemic stroke in which BBB disruption and edema formation are detrimental pathophysiological events unresponsive to steroid treatment. Indeed, mice subjected to transient middle cerebral artery occlusion (tMCAO) and treated with a combination of dexamethasone and the proteasome inhibitor bortezomib 2 h after stroke onset developed significantly (P<0.05) smaller brain infarct volumes at day 1 compared to untreated controls (78.7 ± 19.5 mm³ vs. 43.6 ± 22.3 mm³) **(****Fig. 5A****).** In contrast, both monotherapies failed to show efficacy (P>0.05). The reduction of infarct size was functionally relevant, as the Bederson score assessing global neurological function was significantly better in mice receiving both substances compared to controls or monotherapy (P<0.05). However, the grip test which specifically measures motor function and coordination only showed a non-significant trend towards better outcomes **(****Fig. 5B****).** To further analyze whether the stroke-protective effect observed after dual application of bortezomib and dexamethasone was indeed related to the stabilization of the BBB and reduced edema formation, extravasation of the vascular tracer Evan's blue was determined. Again, dexamethasone or bortezomib alone had no influence on brain edema formation 24 h after tMCAO compared to untreated controls (P>0.05, data not shown), but combination therapy significantly (P<0.05) reduced the amount of extravasal Evan's blue (48.3 ± 16.7 mm³ vs. 18.8 ± 13.2 mm3) **(****Fig. 5C****).**

Then the mouse tMCAO model was used to compare the effects of two commonly used steroids for the treatment of tumour-associated brain edema (dexamethasone and methylprednisolone) and of the endogenous GC cortisol in combination therapy with bortezomib on stroke volume, edema volume and global neurological dysfunction caused by tMCAO **(****Fig. 6****).** Evans blue dye was used as a marker for serum albumin extravasation.

Mice subjected to tMCAO and treated with a combination of dexamethasone and the proteasome inhibitor bortezomib 2 h after stroke onset developed significantly (P<0.05) smaller brain infarct volumes at day 1 compared to untreated controls (78.7 ± 19.5 mm3 vs. 43.6 ± 22.3 mm3) **(****Fig. 5A****).** Also, the combination methylprednisolone and bortezomib led to significant (P<0.05), but not equally strong, reduction in stroke volume (47.6 ± 24.9 mm3). In contrast, treatment with cortisol plus bortezomib did not prove effective in the reduction of stroke Volume compared to the untreated control (78.7 ± 19.5 mm3 vs. 68.4 ± 26.4 mm3). Extravasation of the vascular tracer Evan's blue was evaluated to determine edema volume: reduction in edema volume was equally effective and significant (P<0.02) for both combination therapies, dexamethasone plus bortezomib and methylprednisolone + bortezomib, respectively (18.8 ± 13.2 mm3 vs. 56.6 ± 10.0 mm3 and 14.2 ± 11.6 mm3 vs. 56.6 ± 10.0 mm3) **(****Fig. 6B****).** The Bederson score assessing global neurological function however was clearly best for the combination dexamethasone plus bortezomib (2.1 ± 0.4, P < 0.05), while outome was more inconsistent for the combination methylprednisolone plus bortezomib (2.1 ± 1.1) and did not differ significantly for the combination cortisol plus bortezomib (2.7 ± 1.2) as compared to the untreated control (3.2 ± 0.6) **(****Fig. 6C****).**

### Further References

1. Norris, J.W. 2004. Steroids may have a role in stroke therapy. Stroke 35:228-229.
2. Norris, J.W., and Hachinski, V.C. 1986. High dose steroid treatment in cerebral infarction. Br Med J (Clin Res Ed) 292:21-23.
3. Poungvarin, N. 2004. Steroids have no role in stroke therapy. Stroke 35:229-230.
4. De Reuck, J., Vandekerckhove, T., Bosma, G., De Meulemeester, K., Van Landegem,W., De Waele, J., Tack, E., and De Koninck, J. 1988. Steroid treatment in acute ischaemic stroke. A comparative retrospective study of 556 cases. Eur Neurol 28:70-72.
5. Kumar, N., Jain, S., and Maheshwari, M.C. 1989. Role of dexamethasone in the outcome from acute stroke. J Assoc Physicians India 37:315-317.
6. Beato, M. 1989. Gene regulation by steroid hormones. Cell 56:335-344.
7. Harke, N., Leers, J., Kietz, S., Drenckhahn, D., and Förster, C. 2008. Identification of a distal glucocorticoid responsive element in the occludin gene coding region.. Mol. Cellular Endocrinology 295:39-47.
8. Wallace, A.D., and Cidlowski, J.A. 2001. Proteasome-mediated glucocorticoid receptor degradation restricts transcriptional signaling by glucocorticoids. J Biol Chem 276:42714-42721.
9. Förster, C., Waschke, J., Burek, M., Leers, J., and Drenckhahn, D. 2006. Glucocorticoid effects on mouse microvascular endothelial barrier permeability are brain specific. J Physiol 573:413-425.
10. del Zoppo, G.J., Becker, K.J., and Hallenbeck, J.M. 2001. Inflammation after stroke: is it harmful?-Arch Neurol 58:669-672.
11. Rosenberg, G.A. 2009. Matrix metalloproteinases and their multiple roles in neurodegenerative diseases. Lancet Neurol 8:205-216.
12. Förster, C., Kietz, S., Kahles, T., and Drenckhahn, D. 2007. Dexamethasone induces the expression of metalloproteinase inhibitor TIMP-1 in the murine cerebral vascular endothelial cell line cEND. J. Physiology 580.3:937-949.
13. Förster, C., Burek, M., Romero, I.A., Weksler, B., Couraud, P.O., and Drenckhahn, D. 2008. Differential effects of hydrocortisone and TNF{alpha} on tight junction proteins in an in vitro model of the human blood-brain barrier. J Physiol 586:1937-1949.
14. Romero, I.A., Radewicz, K., Jubin, E., Michel, C.C., Greenwood, J., Couraud, P.O., and Adamson, P. 2003. Changes in cytoskeletal and tight junctional proteins correlate with decreased permeability induced by dexamethasone in cultured rat brain endothelial cells. Neurosci Lett 344:112-116.
15. Blecharz, K., Drenckhahn, D., and Förster, C. 2008. Glucocorticoids increase VEcadherin expression and cause cytoskeletal re-arrangements in murine brain endothelial cEND cells. J Cereb Blood Flow Metab:1139-1149.
16. Koehler, P.J. 1995. Use of corticosteroids in neuro-oncology. Anticancer Drugs 6:19-33.
17. Förster, C., Silwedel, C., Golenhofen, N., Burek, M., Kietz, S., Mankertz, J., and Drenckhahn, D. 2005. Occludin as direct target for glucocorticoid-induced improvement of blood-brain barrier properties in a murine in vitro system. J Physiol 565:475-486.
18. Kim, H., Yoon, S.C., Lee, T.Y., and Jeong, D. 2009. Discriminative cytotoxicity assessment based on various cellular damages. Toxicol Lett 184:13-17.
19. Förster, C. 2008. Tight junctions and the modulation of barrier function in disease. Histochem Cell Biol 130:55-70.
20. D'Atri, F., and Citi, S. 2002. Molecular Complexity of vertebrate tight junctions. Molecular Membrane Biology 19:103-112.
21. Förster, C., Waschke, J., Burek, M., Leers, J., and Drenckhahn, D. 2006. Glucocorticoid Effects on Microvascular Endothelial Barrier Permeability Are Brain Specific. J Physiol 573:413-425.
22. Zhou, J., and Cidlowski, J.A. 2005. The human glucocorticoid receptor: one gene, multiple proteins and diverse responses. Steroids 70:407-417.
23. Leonard, M.O., Godson, C., Brady, H.R., and Taylor, C.T. 2005. Potentiation of glucocorticoid activity in hypoxia through induction of the glucocorticoid receptor. J Immunol 174:2250-2257.
24. Groll, M., and Huber, R. 2004. Inhibitors of the eukaryotic 20S proteasome core particle: a structural approach. Biochim Biophys Acta 1695:33-44.
25. Vinitsky, A., Cardozo, C., Sepp-Lorenzino, L., Michaud, C., and Orlowski, M. 1994. Inhibition of the proteolytic activity of the multicatalytic proteinase complex (proteasome) by substrate-related peptidyl aldehydes. J Biol Chem 269:29860-29866.
26. Benore-Parsons, M., Liebman, J., and Wennogle, L.P. 1991. Binding of glucocorticoid receptors to model DNA response elements. J Cell Biochem 47:330-336.
27. Rubin, L.L., and Staddon, J.M. 1999. The cell biology of the blood-brain barrier. Annu Rev Neurosci 22:11-28.
28. Wolburg, H., and Lippoldt, A. 2002. Tight junctions of the blood-brain barrier: development, composition and regulation. Vascul Pharmacol 38:323-337.
29. Koto, T., Takubo, K., Ishida, S., Shinoda, H., Inoue, M., Tsubota, K., Okada, Y., and Ikeda, E. 2007. Hypoxia disrupts the barrier function of neural blood vessels through changes in the expression of claudin-5 in endothelial cells. Am J Pathol 170:1389-1397.
30. Kago, T., Takagi, N., Date, I., Takenaga, Y., Takagi, K., and Takeo, S. 2006. Cerebral ischemia enhances tyrosine phosphorylation of occludin in brain capillaries. Biochem Biophys Res Commun 339:1197-1203.
31. Kleinschnitz, C., Stoll, G., Bendszus, M., Schuh, K., Pauer, H.U., Burfeind, P., Renne, C., Gailani, D., Nieswandt, B., and Renne, T. 2006. Targeting coagulation factor XII provides protection from pathological thrombosis in cerebral ischemia without interfering with hemostasis. J Exp Med 203:513-518.
32. Austinat, M., Braeuninger, S., Pesquero, J.B., Brede, M., Bader, M., Stoll, G., Renne, T., and Kleinschnitz, C. 2009. Blockade of bradykinin receptor B1 but not bradykinin receptor B2 provides protection from cerebral infarction and brain edema. Stroke 40:285-293.
33. Förster, C., Silwedel, C., Golenhofen, N., Burek, M., Kietz, S., Mankertz, J., and Drenckhahn, D. 2005. Occludin as direct target for glucocorticoid-induced improvement of blood brain-barrier properties in a murine in vitro system. J Physiol 565(Pt 2):475-486.
34. Finley D (2009) Recognition and processing of ubiquitin-protein conjugates by the proteasome. Annu Rev Biochem. 78, 477-513.
35. Kinyamu HK, Chen J, Archer TK (2005) Linking the ubiquitin-proteasome pathway to chromatin remodeling/modification by nuclear receptors. J Mol Endocrinol. 34(2):281-97.

## Claims

1. A combination of
(a) at least one glucocorticoid; and
(ba) at least one proteasome inhibitor and/or
(bb) at least one nucleic acid encoding a glucocorticoid receptor, said glucocorticoid receptor being resistant to proteasomal degradation,
for use in the treatment or prevention of ischemic disorders of the central nervous system.

2. The combination of claim 1, wherein the glucocorticoid is selected from dexamethasone, methylprednisolone and cortisol.

3. The combination of claim 1 or 2, wherein the proteasome inhibitor is selected from peptide boronic acids and esters thereof, lactacystin and analogs thereof, and peptide aldehydes.

4. The combination of any one of claims 1 to 3, wherein the proteasome inhibitor is bortezomib.

5. The combination of any one of the preceding claims, wherein the ischemic disorder of the central nervous system is a cerebral ischemic disorder or a spinal chord ischemia.

6. The combination of any one of the preceding claims, wherein the ischemic disorder of the central nervous system is selected from disruption of the blood brain barrier, cerebral infarct, cerebral stroke, brain edema, hypoxic conditions accompanying traumatic brain injury, ischemic conditions occurring in premature babies, brain hypoperfusion, embolic brain ischemia and thrombotic brain ischemia.

7. The combination of any one of the preceding claims, wherein the glucocorticoid is to be administered prior to, concomitantly with or after the proteasome inhibitor and/or the nucleic acid.

8. The combination of claim 7, wherein the time interval between administration of the glucocorticoid and of the proteasome inhibitor and/or nucleic acid is not to exceed 48, 24, 12, 6, 3, or 0 hours.

9. The combination of any one of the preceding claims, wherein at least the glucocorticoid or at least the proteasome inhibitor and/or the nucleic acid is to be administered within 48, 24, 12, 6, 3, or 0 hours from the ischemic event.

10. At least one proteasome inhibitor and/or at least one nucleic acid encoding a glucocorticoid receptor, said glucocorticoid receptor being resistant to proteasomal degradation, for use in treating or preventing glucocorticoid resistance in ischemic disorders of the central nervous system.

11. The proteasome inhibitor, or the proteasome inhibitor and the nucleic acid of claim 10, wherein said proteasome inhibitor is as defined in claim 3 or 4.

12. The proteasome inhibitor and/or the nucleic acid of claim 10 or 11, wherein the ischemic disorder is as defined in claim 5 or 6.

13. The combination of any one of claims 1 to 9, or the proteasome inhibitor and/or the nucleic acid of any one of claim 10 to 12, wherein said glucocorticoid receptor being resistant to proteasomal degradation is a glucocorticoid receptor wherein residues important for ubiquitin conjugation are mutated such that ubiquitin conjugation is abolished or substantially decreased.

14. The combination of any one of claims 1 to 9 or 13 or the proteasome inhibitor and/or the nucleic acid of any one of claim 10 to 13, wherein said glucocorticoid receptor being resistant to proteasomal degradation is
(i) a mutant of the human glucocorticoid receptor, the mutant differing from the wild type in that K419 is replaced with a different amino acid residue, or
(ii) a mutant of a glucocorticoid receptor of a different mammalian species, said mutant of a glucocorticoid receptor of a different mammalian species differing from the corresponding wild type glucocorticoid receptor in that the lysine corresponding to position 419 of the human glucocorticoid receptor is replaced with a different amino acid residue.


SEQUENCE LISTING

<110> Julius-Maximilians-Universität Würzburg

<120> Means and methods for treating ischemic conditions

<130> R2527 EP

<160> 4

<170> PatentIn version 3.4

<210> 1
<211> 7286
<212> DNA
<213> homo sapiens

<400> 1
aggttatgta agggtttgct ttcaccccat tcaaaaggta cctcttcctc ttctcttgct 60

ccctctcgcc ctcattcttg tgcctatgca gacatttgag tagaggcgaa tcactttcac 120

ttctgctggg gaaattgcaa cacgcttctt taaatggcag agagaaggag aaaacttaga 180

tcttctgata ccaaatcact ggaccttaga aggtcagaaa tctttcaagc cctgcaggac 240

cgtaaaatgc gcatgtgtcc aacggaagca ctggggcatg agtggggaag gaatagaaac 300

agaaagaggg taagagaaga aaaaagggaa agtggtgaag gcagggagga aaattgctta 360

gtgtgaatat gcacgcattc atttagtttt caaatccttg ttgagcatga taaaattccc 420

agcatcagac ctcacatgtt ggtttccatt aggatctgcc tgggggaata tctgctgaat 480

cagtggctct gagctgaact aggaaattca ccataattag gagagtcact gtatttctct 540

ccaaaaaaaa aaaagttata cccgagagac aggatcttct gatctgaaat tttcttcact 600

tctgaaattc tctggtttgt gctcatcgtt ggtagctatt tgttcatcaa gagttgtgta 660

gctggcttct tctgaaaaaa ggaatctgcg tcatatctaa gtcagatttc attctggtgc 720

tctcagagca gttagcccag gaaaggggcc agcttctgtg acgactgctg cagaggcagg 780

tgcagtttgt gtgccacaga tattaacttt gataagcact taatgagtgc cttctctgtg 840

cgagaatggg gaggaacaaa atgcagctcc taccctcctc gggctttagt tgtaccttaa 900

taacaggaat tttcatctgc ctggctcctt tcctcaaaga acaaagaaga ctttgcttca 960

ttaaagtgtc tgagaaggaa gttgatattc actgatggac tccaaagaat cattaactcc 1020

tggtagagaa gaaaacccca gcagtgtgct tgctcaggag aggggagatg tgatggactt 1080

ctataaaacc ctaagaggag gagctactgt gaaggtttct gcgtcttcac cctcactggc 1140

tgtcgcttct caatcagact ccaagcagcg aagacttttg gttgattttc caaaaggctc 1200

agtaagcaat gcgcagcagc cagatctgtc caaagcagtt tcactctcaa tgggactgta 1260

tatgggagag acagaaacaa aagtgatggg aaatgacctg ggattcccac agcagggcca 1320

aatcagcctt tcctcggggg aaacagactt aaagcttttg gaagaaagca ttgcaaacct 1380

caataggtcg accagtgttc cagagaaccc caagagttca gcatccactg ctgtgtctgc 1440

tgcccccaca gagaaggagt ttccaaaaac tcactctgat gtatcttcag aacagcaaca 1500

tttgaagggc cagactggca ccaacggtgg caatgtgaaa ttgtatacca cagaccaaag 1560
cacctttgac attttgcagg atttggagtt ttcttctggg tccccaggta aagagacgaa 1620
tgagagtcct tggagatcag acctgttgat agatgaaaac tgtttgcttt ctcctctggc 1680
gggagaagac gattcattcc ttttggaagg aaactcgaat gaggactgca agcctctcat 1740
tttaccggac actaaaccca aaattaagga taatggagat ctggttttgt caagccccag 1800
taatgtaaca ctgccccaag tgaaaacaga aaaagaagat ttcatcgaac tctgcacccc 1860
tggggtaatt aagcaagaga aactgggcac agtttactgt caggcaagct ttcctggagc 1920
aaatataatt ggtaataaaa tgtctgccat ttctgttcat ggtgtgagta cctctggagg 1980
acagatgtac cactatgaca tgaatacagc atccctttct caacagcagg atcagaagcc 2040
tatttttaat gtcattccac caattcccgt tggttccgaa aattggaata ggtgccaagg 2100
atctggagat gacaacttga cttctctggg gactctgaac ttccctggtc gaacagtttt 2160
ttctaatggc tattcaagcc ccagcatgag accagatgta agctctcctc catccagctc 2220
ctcaacagca acaacaggac cacctcccgc actctgcctg gtgtgctctg atgaagcttc 2280
aggatgtcat tatggagtct taacttgtgg aagctgtaaa gttttcttca aaagagcagt 2340
ggaaggacag cacaattacc tatgtgctgg aaggaatgat tgcatcatcg ataaaattcg 2400
aagaaaaaac tgcccagcat gccgctatcg aaaatgtctt caggctggaa tgaacctgga 2460
agctcgaaaa acaaagaaaa aaataaaagg aattcagcag gccactacag gagtctcaca 2520
agaaacctct gaaaatcctg gtaacaaaac aatagttcct gcaacgttac cacaactcac 2580
ccctaccctg gtgtcactgt tggaggttat tgaacctgaa gtgttatatg caggatatga 2640
tagctctgtt ccagactcaa cttggaggat catgactacg ctcaacatgt taggagggcg 2700
gcaagtgatt gcagcagtga aatgggcaaa ggcaatacca ggtttcagga acttacacct 2760
ggatgaccaa atgaccctac tgcagtactc ctggatgttt cttatggcat ttgctctggg 2820
gtggagatca tatagacaat caagtgcaaa cctgctgtgt tttgctcctg atctgattat 2880
taatgagcag agaatgactc taccctgcat gtacgaccaa tgtaaacaca tgctgtatgt 2940
ttcctctgag ttacacaggc ttcaggtatc ttatgaagag tatctctgta tgaaaacctt 3000
actgcttctc tcttcagttc ctaaggacgg tctgaagagc caagagctat ttgatgaaat 3060
tagaatgacc tacatcaaag agctaggaaa agccattgtc aagagggaag gaaactccag 3120
ccagaactgg cagcggtttt atcaactgac aaaactcttg gattctatgc atgaagtggt 3180
tgaaaatctc cttaactatt gcttccaaac atttttggat aagaccatga gtattgaatt 3240
ccccgagatg ttagctgaaa tcatcaccaa tcagatacca aaatattcaa atggaaatat 3300
caaaaaactt ctgtttcatc aaaagtgact gccttaataa gaatggttgc cttaaagaaa 3360
gtcgaattaa tagcttttat tgtataaact atcagtttgt cctgtagagg ttttgttgtt 3420
ttatttttta ttgttttcat ctgttgtttt gttttaaata cgcactacat gtggtttata 3480
gagggccaag acttggcaac agaagcagtt gagtcgtcat cacttttcag tgatgggaga 3540

gtagatggtg aaatttatta gttaatatat cccagaaatt agaaacctta atatgtggac 3600
gtaatctcca cagtcaaaga aggatggcac ctaaaccacc agtgcccaaa gtctgtgtga 3660
tgaactttct cttcatactt tttttcacag ttggctggat gaaattttct agactttctg 3720
ttggtgtatc ccccccctgt atagttagga tagcattttt gatttatgca tggaaacctg 3780
aaaaaaagtt tacaagtgta tatcagaaaa gggaagttgt gccttttata gctattactg 3840
tctggtttta acaatttcct ttatatttag tgaactacgc ttgctcattt tttcttacat 3900
aattttttat tcaagttatt gtacagctgt ttaagatggg cagctagttc gtagctttcc 3960
caaataaact ctaaacatta atcaatcatc tgtgtgaaaa tgggttggtg cttctaacct 4020
gatggcactt agctatcaga agaccacaaa aattgactca aatctccagt attcttgtca 4080
aaaaaaaaaa aaaaaaagct catattttgt atatatctgc ttcagtggag aattatatag 4140
gttgtgcaaa ttaacagtcc taactggtat agagcaccta gtccagtgac ctgctgggta 4200
aactgtggat gatggttgca aaagactaat ttaaaaaata actaccaaga ggccctgtct 4260
gtacctaacg ccctattttt gcaatggcta tatggcaaga aagctggtaa actatttgtc 4320
tttcaggacc ttttgaagta gtttgtataa cttcttaaaa gttgtgattc cagataacca 4380
gctgtaacac agctgagaga cttttaatca gacaaagtaa ttcctctcac taaactttac 4440
ccaaaaacta aatctctaat atggcaaaaa tggctagaca cccattttca cattcccatc 4500
tgtcaccaat tggttaatct ttcctgatgg tacaggaaag ctcagctact gatttttgtg 4560
atttagaact gtatgtcaga catccatgtt tgtaaaacta cacatcccta atgtgtgcca 4620
tagagtttaa cacaagtcct gtgaatttct tcactgttga aaattatttt aaacaaaata 4680
gaagctgtag tagccctttc tgtgtgcacc ttaccaactt tctgtaaact caaaacttaa 4740
catatttact aagccacaag aaatttgatt tctattcaag gtggccaaat tatttgtgta 4800
atagaaaact gaaaatctaa tattaaaaat atggaacttc taatatattt ttatatttag 4860
ttatagtttc agatatatat catattggta ttcactaatc tgggaaggga agggctactg 4920
cagctttaca tgcaatttat taaaatgatt gtaaaatagc ttgtatagtg taaaataaga 4980
atgattttta gatgagattg ttttatcatg acatgttata tattttttgt aggggtcaaa 5040
gaaatgctga tggataacct atatgattta tagtttgtac atgcattcat acaggcagcg 5100
atggtctcag aaaccaaaca gtttgctcta ggggaagagg gagatggaga ctggtcctgt 5160
gtgcagtgaa ggttgctgag gctctgaccc agtgagatta cagaggaagt tatcctctgc 5220
ctcccattct gaccaccctt ctcattccaa cagtgagtct gtcagcgcag gtttagttta 5280
ctcaatctcc ccttgcacta aagtatgtaa agtatgtaaa caggagacag gaaggtggtg 5340
cttacatcct taaaggcacc atctaatagc gggttacttt cacatacagc cctcccccag 5400
cagttgaatg acaacagaag cttcagaagt ttggcaatag tttgcataga ggtaccagca 5460
atatgtaaat agtgcagaat ctcataggtt gccaataata cactaattcc tttctatcct 5520
acaacaagag tttatttcca aataaaatga ggacatgttt ttgttttctt tgaatgcttt 5580

ttgaatgtta tttgttattt tcagtatttt ggagaaatta tttaataaaa aaacaatcat 5640
ttgctttttg aatgctctct aaaagggaat gtaatatttt aagatggtgt gtaacccggc 5700
tggataaatt tttggtgcct aagaaaactg cttgaatatt cttatcaatg acagtgttaa 5760
gtttcaaaaa gagcttctaa aacgtagatt atcattcctt tatagaatgt tatgtggtta 5820
aaaccagaaa gcacatctca cacattaatc tgattttcat cccaacaatc ttggcgctca 5880
aaaaatagaa ctcaatgaga aaaagaagat tatgtgcact tcgttgtcaa taataagtca 5940
actgatgctc atcgacaact ataggaggct tttcattaaa tgggaaaaga agctgtgccc 6000
ttttaggata cgtgggggaa aagaaagtca tcttaattat gtttaattgt ggatttaagt 6060
gctatatggt ggtgctgttt gaaagcagat ttatttccta tgtatgtgtt atctggccat 6120
cccaacccaa actgttgaag tttgtagtaa cttcagtgag agttggttac tcacaacaaa 6180
tcctgaaaag tatttttagt gtttgtaggt attctgtggg atactataca agcagaactg 6240
aggcacttag gacataacac ttttggggta tatatatcca aatgcctaaa actatgggag 6300
gaaaccttgg ccaccccaaa aggaaaacta acatgatttg tgtctatgaa gtgctggata 6360
attagcatgg gatgagctct gggcatgcca tgaaggaaag ccacgctccc ttcagaattc 6420
agaggcaggg agcaattcca gtttcaccta agtctcataa ttttagttcc cttttaaaaa 6480
ccctgaaaac tacatcacca tggaatgaaa aatattgtta tacaatacat tgatctgtca 6540
aacttccaga accatggtag ccttcagtga gatttccatc ttggctggtc actccctgac 6600
tgtagctgta ggtgaatgtg tttttgtgtg tgtgtgtctg gttttagtgt cagaagggaa 6660
ataaaagtgt aaggaggaca ctttaaaccc tttgggtgga gtttcgtaat ttcccagact 6720
attttcaagc aacctggtcc acccaggatt agtgaccagg ttttcaggaa aggatttgct 6780
tctctctaga aaatgtctga aaggatttta ttttctgatg aaaggctgta tgaaaatacc 6840
ctcctcaaat aacttgctta actacatata gattcaagtg tgtcaatatt ctattttgta 6900
tattaaatgc tatataatgg ggacaaatct atattatact gtgtatggca ttattaagaa 6960
gctttttcat tattttttat cacagtaatt ttaaaatgtg taaaaattaa aaccagtgac 7020
tcctgtttaa aaataaaagt tgtagttttt tattcatgct gaataataat ctgtagttaa 7080
aaaaaaagtg tctttttacc tacgcagtga aatgtcagac tgtaaaacct tgtgtggaaa 7140
tgtttaactt ttattttttc atttaaattt gctgttctgg tattaccaaa ccacacattt 7200
gtaccgaatt ggcagtaaat gttagccatt tacagcaatg ccaaatatgg agaaacatca 7260
taataaaaaa atctgctttt tcatta 7286

<210> 2
<211> 777
<212> PRT
<213> homo sapiens

<400> 2

Met Asp Ser Lys Glu Ser Leu Thr Pro Gly Arg Glu Glu Asn Pro Ser
1               5                   10                  15

Ser Val Leu Ala Gln Glu Arg Gly Asp Val Met Asp Phe Tyr Lys Thr
            20                  25                  30

Leu Arg Gly Gly Ala Thr Val Lys Val Ser Ala Ser Ser Pro Ser Leu
        35                  40                  45

Ala Val Ala Ser Gln Ser Asp Ser Lys Gln Arg Arg Leu Leu Val Asp
    50                  55                  60

Phe Pro Lys Gly Ser Val Ser Asn Ala Gln Gln Pro Asp Leu Ser Lys
65                  70                  75                  80

Ala Val Ser Leu Ser Met Gly Leu Tyr Met Gly Glu Thr Glu Thr Lys
                85                  90                  95

Val Met Gly Asn Asp Leu Gly Phe Pro Gln Gln Gly Gln Ile Ser Leu
            100                 105                 110

Ser Ser Gly Glu Thr Asp Leu Lys Leu Leu Glu Glu Ser Ile Ala Asn
        115                 120                 125

Leu Asn Arg Ser Thr Ser Val Pro Glu Asn Pro Lys Ser Ser Ala Ser
    130                 135                 140

Thr Ala Val Ser Ala Ala Pro Thr Glu Lys Glu Phe Pro Lys Thr His
145                 150                 155                 160

Ser Asp Val Ser Ser Glu Gln Gln His Leu Lys Gly Gln Thr Gly Thr
                165                 170                 175

Asn Gly Gly Asn Val Lys Leu Tyr Thr Thr Asp Gln Ser Thr Phe Asp
            180                 185                 190

Ile Leu Gln Asp Leu Glu Phe Ser Ser Gly Ser Pro Gly Lys Glu Thr
        195                 200                 205

Asn Glu Ser Pro Trp Arg Ser Asp Leu Leu Ile Asp Glu Asn Cys Leu
    210                 215                 220

Leu Ser Pro Leu Ala Gly Glu Asp Asp Ser Phe Leu Leu Glu Gly Asn
225                 230                 235                 240

Ser Asn Glu Asp Cys Lys Pro Leu Ile Leu Pro Asp Thr Lys Pro Lys
                245                 250                 255

Ile Lys Asp Asn Gly Asp Leu Val Leu Ser Ser Pro Ser Asn Val Thr
            260                 265                 270

Leu Pro Gln Val Lys Thr Glu Lys Glu Asp Phe Ile Glu Leu Cys Thr
        275                 280                 285

Pro Gly Val Ile Lys Gln Glu Lys Leu Gly Thr Val Tyr Cys Gln Ala
    290                 295                 300

Ser Phe Pro Gly Ala Asn Ile Ile Gly Asn Lys Met Ser Ala Ile Ser
305                 310                 315                 320

Val His Gly Val Ser Thr Ser Gly Gly Gln Met Tyr His Tyr Asp Met
                325                 330                 335

Asn Thr Ala Ser Leu Ser Gln Gln Gln Asp Gln Lys Pro Ile Phe Asn
            340                 345                 350

Val Ile Pro Pro Ile Pro Val Gly Ser Glu Asn Trp Asn Arg Cys Gln
        355                 360                 365

Gly Ser Gly Asp Asp Asn Leu Thr Ser Leu Gly Thr Leu Asn Phe Pro
    370                 375                 380

Gly Arg Thr Val Phe Ser Asn Gly Tyr Ser Ser Pro Ser Met Arg Pro
385                 390                 395                 400

Asp Val Ser Ser Pro Pro Ser Ser Ser Ser Thr Ala Thr Thr Gly Pro
                405                 410                 415

Pro Pro Ala Leu Cys Leu Val Cys Ser Asp Glu Ala Ser Gly Cys His
            420                 425                 430

Tyr Gly Val Leu Thr Cys Gly Ser Cys Lys Val Phe Phe Lys Arg Ala
        435                 440                 445

Val Glu Gly Gln His Asn Tyr Leu Cys Ala Gly Arg Asn Asp Cys Ile
    450                 455                 460

Ile Asp Lys Ile Arg Arg Lys Asn Cys Pro Ala Cys Arg Tyr Arg Lys
465                 470                 475                 480

Cys Leu Gln Ala Gly Met Asn Leu Glu Ala Arg Lys Thr Lys Lys Lys
                485                 490                 495

Ile Lys Gly Ile Gln Gln Ala Thr Thr Gly Val Ser Gln Glu Thr Ser
            500                 505                 510

Glu Asn Pro Gly Asn Lys Thr Ile Val Pro Ala Thr Leu Pro Gln Leu
        515                 520                 525

Thr Pro Thr Leu Val Ser Leu Leu Glu Val Ile Glu Pro Glu Val Leu
    530                 535                 540

Tyr Ala Gly Tyr Asp Ser Ser Val Pro Asp Ser Thr Trp Arg Ile Met
545                 550                 555                 560

Thr Thr Leu Asn Met Leu Gly Gly Arg Gln Val Ile Ala Ala Val Lys
                565                 570                 575

Trp Ala Lys Ala Ile Pro Gly Phe Arg Asn Leu His Leu Asp Asp Gln
            580                 585                 590

Met Thr Leu Leu Gln Tyr Ser Trp Met Phe Leu Met Ala Phe Ala Leu
        595                 600                 605

Gly Trp Arg Ser Tyr Arg Gln Ser Ser Ala Asn Leu Leu Cys Phe Ala
    610                 615                 620

Pro Asp Leu Ile Ile Asn Glu Gln Arg Met Thr Leu Pro Cys Met Tyr
625                 630                 635                 640

Asp Gln Cys Lys His Met Leu Tyr Val Ser Ser Glu Leu His Arg Leu
                645                 650                 655

Gln Val Ser Tyr Glu Glu Tyr Leu Cys Met Lys Thr Leu Leu Leu Leu
            660                 665                 670

Ser Ser Val Pro Lys Asp Gly Leu Lys Ser Gln Glu Leu Phe Asp Glu
        675                 680                 685

Ile Arg Met Thr Tyr Ile Lys Glu Leu Gly Lys Ala Ile Val Lys Arg
    690                 695                 700

Glu Gly Asn Ser Ser Gln Asn Trp Gln Arg Phe Tyr Gln Leu Thr Lys
705                 710                 715                 720

Leu Leu Asp Ser Met His Glu Val Val Glu Asn Leu Leu Asn Tyr Cys
                725                 730                 735

Phe Gln Thr Phe Leu Asp Lys Thr Met Ser Ile Glu Phe Pro Glu Met
            740                 745                 750

Leu Ala Glu Ile Ile Thr Asn Gln Ile Pro Lys Tyr Ser Asn Gly Asn
        755                 760                 765

Ile Lys Lys Leu Leu Phe His Gln Lys
    770                 775

<210>  3
<211>  2352
<212>  DNA
<213>  mus musculus

<400>  3
atggactcca aagaatcctt agctccccct ggtagagacg aagtccccag cagtttgctt      60

ggccggggga ggggaagcgt gatggacttg tataaaaccc tgaggggtgg agctacagtc     120

aaggtttctg cgtcttcacc ctcagtggct gctgcttctc aggcagattc caagcagcag 180
aggattctcc ttgatttttc aaaaggctca gcaagcaatg cacagcagca gcagcagcag 240
cagcagccgc agccagattt atccaaagcc gtttcactgt ccatgggact gtatatggga 300
gagaccgaaa caaaagtgat ggggaatgac ttgggctacc cacagcaggg ccagcttggc 360
ctctcctctg gggaaacaga ctttcggctt ctggaagaaa gcattgcaaa cctcaatagg 420
tcgaccagcc gtccagagaa tcccaagagt tcaacacctg cagctgggtg tgctaccccg 480
acagagaagg agtttcccca gactcactct gatccatctt cagaacagca aaatagaaaa 540
agccagcctg gcaccaacgg tggcagtgtg aaattgtata ccacagacca aagcaccttt 600
gacatcttgc aggatttgga gttttctgcc gggtccccag gtaaagagac aaacgagagt 660
ccttggaggt cagacctgtt gatagatgaa aacttgcttt ctcctttggc gggagaagat 720
gatccattcc ttctggaagg ggacgtgaat gaggattgca agcctcttat tttaccggac 780
actaaaccta aaattcagga tactggagat acaatcttat caagccccag cagtgtggca 840
ctgccccaag tgaaaacaga gaaagatgat ttcattgagc tttgcacccc tggggtaatt 900
aagcaagaga aactgggccc ggtttattgc caggcaagct tttctgggac aaatataatt 960
gggaataaaa tgtctgccat ttctgttcat ggcgtgagta cctctggagg acagatgtac 1020
cactatgaca tgaatacagc atccctttct cagcagcagg atcagaagcc tgtttttaat 1080
gtcattccac caattcctgt tggttctgaa aactggaata ggtgccaagg gtctggagag 1140
gacaacctga cttccttggg ggctatgaac ttcgcaggcc gctcagtgtt ttctaatgga 1200
tattcaagcc ctggaatgag accagatgtg agttctcctc cgtccagctc ctccacagca 1260
acgggaccac ctcccgcact ctgcctggtg tgctccgatg aagcttcggt atgccattat 1320
ggggtgctga cgtgtggaag ctgtaaagtc ttctttaaaa gagcagtgga aggacagcac 1380
aattaccttt gtgctggaag aaatgattgc atcattgata aaattcgaag aaaaaactgt 1440
ccagcatgcc gctatcgaaa atgtcttcaa gctggaatga acctggaagc tcgaaaaacg 1500
aagaaaaaaa ttaaaggaat tcagcaagcc actgcaggag tctcacaaga cacttctgaa 1560
aacgctaaca aaacaatagt tcctgccgcg ctgccacagc ttacccctac cctggtgtca 1620
ctgctggagg tgatcgagcc tgaggtgtta tatgcaggat atgacagctc tgttccagac 1680
tcagcatgga gaattatgac cacgctcaac atgttaggtg ggcgccaagt gattgccgca 1740
gtgaaatggg caaaggcgat accaggattc agaaacttac acctggatga ccaaatgacc 1800
cttctacagt actcatggat gtttctcatg gcatttgccc tgggttggag atcatacaga 1860
caagcaagtg gaaacctgct atgctttgct cctgatctga ttattaatga gcagagaatg 1920
actctaccct gcatgtatga ccaatgtaaa cacatgctgt ttatctccac tgaattacaa 1980
agattgcagg tatcctatga agagtatctc tgtatgaaaa ccttactgct tctctcctca 2040
gttcctaagg aaggtctgaa gagccaagag ttatttgatg agattcgaat gacttatatc 2100
aaagagctag gaaaagccat tgtcaaaagg gaaggaaact ccagtcagaa ttggcagcgg 2160

ttttatcaac tgacaaaact tttggactcc atgcatgatg tggttgaaaa tctccttagc 2220

tactgcttcc aaacattttt ggataagtcc atgagtattg aattcccaga gatgttagct 2280

gaaatcatca ctaatcagat accaaaatac tcaaatggaa atatcaaaaa gcttctgttt 2340

catcagaaat ga 2352

<210> 4
<211> 783
<212> PRT
<213> mus musculus

<400> 4

Met Asp Ser Lys Glu Ser Leu Ala Pro Pro Gly Arg Asp Glu Val Pro
1               5                   10                  15

Ser Ser Leu Leu Gly Arg Gly Arg Gly Ser Val Met Asp Leu Tyr Lys
            20                  25                  30

Thr Leu Arg Gly Gly Ala Thr Val Lys Val Ser Ala Ser Ser Pro Ser
        35                  40                  45

Val Ala Ala Ala Ser Gln Ala Asp Ser Lys Gln Gln Arg Ile Leu Leu
    50                  55                  60

Asp Phe Ser Lys Gly Ser Ala Ser Asn Ala Gln Gln Gln Gln Gln Gln
65                  70                  75                  80

Gln Gln Pro Gln Pro Asp Leu Ser Lys Ala Val Ser Leu Ser Met Gly
                85                  90                  95

Leu Tyr Met Gly Glu Thr Glu Thr Lys Val Met Gly Asn Asp Leu Gly
            100                 105                 110

Tyr Pro Gln Gln Gly Gln Leu Gly Leu Ser Ser Gly Glu Thr Asp Phe
        115                 120                 125

Arg Leu Leu Glu Glu Ser Ile Ala Asn Leu Asn Arg Ser Thr Ser Arg
    130                 135                 140

Pro Glu Asn Pro Lys Ser Ser Thr Pro Ala Ala Gly Cys Ala Thr Pro
145                 150                 155                 160

Thr Glu Lys Glu Phe Pro Gln Thr His Ser Asp Pro Ser Ser Glu Gln
                165                 170                 175

Gln Asn Arg Lys Ser Gln Pro Gly Thr Asn Gly Gly Ser Val Lys Leu
            180                 185                 190

Tyr Thr Thr Asp Gln Ser Thr Phe Asp Ile Leu Gln Asp Leu Glu Phe
        195                 200                 205

Ser Ala Gly Ser Pro Gly Lys Glu Thr Asn Glu Ser Pro Trp Arg Ser
    210                 215                 220

Asp Leu Leu Ile Asp Glu Asn Leu Leu Ser Pro Leu Ala Gly Glu Asp
225                 230                 235                 240

Asp Pro Phe Leu Leu Glu Gly Asp Val Asn Glu Asp Cys Lys Pro Leu
                245                 250                 255

Ile Leu Pro Asp Thr Lys Pro Lys Ile Gln Asp Thr Gly Asp Thr Ile
            260                 265                 270

Leu Ser Ser Pro Ser Ser Val Ala Leu Pro Gln Val Lys Thr Glu Lys
        275                 280                 285

Asp Asp Phe Ile Glu Leu Cys Thr Pro Gly Val Ile Lys Gln Glu Lys
    290                 295                 300

Leu Gly Pro Val Tyr Cys Gln Ala Ser Phe Ser Gly Thr Asn Ile Ile
305                 310                 315                 320

Gly Asn Lys Met Ser Ala Ile Ser Val His Gly Val Ser Thr Ser Gly
                325                 330                 335

Gly Gln Met Tyr His Tyr Asp Met Asn Thr Ala Ser Leu Ser Gln Gln
            340                 345                 350

Gln Asp Gln Lys Pro Val Phe Asn Val Ile Pro Pro Ile Pro Val Gly
        355                 360                 365

Ser Glu Asn Trp Asn Arg Cys Gln Gly Ser Gly Glu Asp Asn Leu Thr
    370                 375                 380

Ser Leu Gly Ala Met Asn Phe Ala Gly Arg Ser Val Phe Ser Asn Gly
385                 390                 395                 400

Tyr Ser Ser Pro Gly Met Arg Pro Asp Val Ser Ser Pro Pro Ser Ser
                405                 410                 415

Ser Ser Thr Ala Thr Gly Pro Pro Pro Ala Leu Cys Leu Val Cys Ser
            420                 425                 430

Asp Glu Ala Ser Val Cys His Tyr Gly Val Leu Thr Cys Gly Ser Cys
        435                 440                 445

Lys Val Phe Phe Lys Arg Ala Val Glu Gly Gln His Asn Tyr Leu Cys
    450                 455                 460

Ala Gly Arg Asn Asp Cys Ile Ile Asp Lys Ile Arg Arg Lys Asn Cys
465                 470                 475                 480

Pro Ala Cys Arg Tyr Arg Lys Cys Leu Gln Ala Gly Met Asn Leu Glu
                485                 490                 495

Ala Arg Lys Thr Lys Lys Lys Ile Lys Gly Ile Gln Gln Ala Thr Ala
            500                 505                 510

Gly Val Ser Gln Asp Thr Ser Glu Asn Ala Asn Lys Thr Ile Val Pro
        515                 520                 525

Ala Ala Leu Pro Gln Leu Thr Pro Thr Leu Val Ser Leu Leu Glu Val
    530                 535                 540

Ile Glu Pro Glu Val Leu Tyr Ala Gly Tyr Asp Ser Ser Val Pro Asp
545                 550                 555                 560

Ser Ala Trp Arg Ile Met Thr Thr Leu Asn Met Leu Gly Gly Arg Gln
                565                 570                 575

Val Ile Ala Ala Val Lys Trp Ala Lys Ala Ile Pro Gly Phe Arg Asn
            580                 585                 590

Leu His Leu Asp Asp Gln Met Thr Leu Leu Gln Tyr Ser Trp Met Phe
        595                 600                 605

Leu Met Ala Phe Ala Leu Gly Trp Arg Ser Tyr Arg Gln Ala Ser Gly
    610                 615                 620

Asn Leu Leu Cys Phe Ala Pro Asp Leu Ile Ile Asn Glu Gln Arg Met
625                 630                 635                 640

Thr Leu Pro Cys Met Tyr Asp Gln Cys Lys His Met Leu Phe Ile Ser
                645                 650                 655

Thr Glu Leu Gln Arg Leu Gln Val Ser Tyr Glu Glu Tyr Leu Cys Met
            660                 665                 670

Lys Thr Leu Leu Leu Leu Ser Ser Val Pro Lys Glu Gly Leu Lys Ser
        675                 680                 685

Gln Glu Leu Phe Asp Glu Ile Arg Met Thr Tyr Ile Lys Glu Leu Gly
    690                 695                 700

Lys Ala Ile Val Lys Arg Glu Gly Asn Ser Ser Gln Asn Trp Gln Arg
705                 710                 715                 720

Phe Tyr Gln Leu Thr Lys Leu Leu Asp Ser Met His Asp Val Val Glu
                725                 730                 735

Asn Leu Leu Ser Tyr Cys Phe Gln Thr Phe Leu Asp Lys Ser Met Ser
            740                 745                 750

Ile Glu Phe Pro Glu Met Leu Ala Glu Ile Ile Thr Asn Gln Ile Pro
        755                 760                 765

Lys Tyr Ser Asn Gly Asn Ile Lys Lys Leu Leu Phe His Gln Lys
    770                 775                 780